# EUROPEAN PATENT SPECIFICATION

(11) **EP 1 301 146 B1**
(45) Date of publication and mention of the grant of the patent: **02.06.2010**
(21) Application number: 01956749.4
(22) Date of filing: 20.07.2001
(51) Int. Cl.: A61F 2/06

(54) **ARTIFICIAL VASCULAR GRAFTS AND METHODS OF PRODUCING AND USING SAME**
KÜNSTLICHES, VASKULÄRES TRANSPLANTAT SOWIE VERFAHREN ZU SEINER HERSTELLUNG UND SEINEM GEBRAUCH
GREFFONS VASCULAIRES ARTIFICIELS ET PROCEDES DE FABRICATION ET D'UTILISATION CORRESPONDANTS

(30) Priority: 20.07.2000 US 620227
(43) Date of publication of application: 16.04.2003
(73) Proprietor: M.G.V.S. Ltd., 34362 Haifa (IL)
(72) Inventor: FLUGELMAN, Moshe, Y., 34642 Haifa (IL); PREIS, Meir, 34402 Haifa (IL); GLUZMAN, Zoya, 25167 Tal-El (IL); KOREN, Belly, 20692 Yokneam Ilit (IL); WEISZ, Anat, 34679 Haifa (IL); COHEN, Tzafra, 34521 Haifa (IL)
(74) Representative: Machtalère, Georges
(86) International application number: PCT/IL2001/000670
(87) International publication number: WO 2002/007646

(56) References cited:
- WO-A-00/64377
- WO-A-95/25547
- US-A- 5 631 153
- US-A- 5 674 722
- US-A- 5 700 346
- US-A- 5 785 965
- US-A- 6 010 573
- DARDIK A ET AL: "Chronic in vitro shear stress stimulates endothelial cell retention on prosthetic vascular grafts and reduces subsequent in vivo neointimal thickness" JOURNAL OF VASCULAR SURGERY, C.V. MOSBY CO., ST. LOUIS, MO, US, vol. 29, no. 1, 1 January 1999 (1999-01-01), pages 157-167, XP022849125 ISSN: 0741-5214 [retrieved on 1999-01-01]

## Description

### FIELD AND BACKGROUND OF THE INVENTION

The present invention relates to artificial vascular grafts. More particularly, the present invention relates to genetically transformed cells and methods of generating same, which cells are useful in the generation of improved artificial vascular grafts. The present invention further relates to methods of using the artificial vascular grafts for therapy of vascular diseases.

### Vascular diseases:

Vascular diseases are a major cause of morbidity and mortality in the developed world and thus affects a large part of the population. Bypass surgery utilizing venous, arterial or synthetic conduit grafts is often used to alleviate the clinical sequelae of these diseases by allowing or restoring blood flow around a blocked or damaged blood vessel. It is estimated that over 1 million such procedures are performed in developed countries annually and that 30-50% of these interventions fail within 5 to 7 years (1-4).

### Arterial grafts:

Arterial grafts have a higher durability rate in the long term when compared to venous and synthetic grafts (5). Yet, arterial grafts can be used only in limited clinical circumstances such as, for example, aorto-coronary bypass surgery. There is an ever-increasing need for synthetic grafts with a biological-compatible surface of high durability which can be used for extended periods of time. The demand for small caliber grafts is especially high in lesions below the femoral artery and in patients who have limited native vessels such as with varicose veins or previous bypass surgery (2,3). Use of polytetrafluoroethylene (PTFE) and other materials in synthetic grafts has gained popularity due to the biocompatibility of the material itself and to its durability. Modification of the synthetic material was described in many animal studies and in a few human studies (5-10).

To improve function and durability of synthetic grafts, autologous endothelial cells (EC) have been used to seed grafts before implantation. Endothelial cells provide the best biocompatible surface, and may provide long term protection from thrombosis due to their thrombolytic capacity (11,12). In addition, EC coverage can also prevent neointimal proliferation and inflammatory reaction in the graft (13). However, to date, randomized large scale studies have not proven that EC seeding can improve graft durability (8-10).

Endothelial cells used for seeding grafts before implantation are typically harvested from short venous segments or from adipose tissue. Other sources may be cells from transgenic animals having human characteristics or autologous EC from the individual patient. Another possible source for EC is progenitor cells isolated from the peripheral blood or bone marrow.

New strategies have been attempted at the stage of artificial graft design and preparation in order to improve efficiency of artificial vascular grafts utilized in treating human vascular diseases. Complete endothelialization of artificial vascular graft luminal surface is still extremely delayed and has a major impact on graft patency. Therefor, a major task is to improve both graft coverage and cell adherence to the graft matrix. Previous attempts in which EC were seeded onto synthetic grafts, resulted in incomplete endothelialization as well as cell detachment upon exposure to blood flow conditions.

One of the most recent techniques of improving graft endothelialization is to coat it with genetically transformed cells having better proliferating capacity (see, for example, U.S. Pat. No. 5,785,965).

To enhance differentiation of progenitor bone marrow cells and provide a short and reliable method for generating EC useable in artificial graft preparation, such cells are typically transformed with a vascular endothelial growth factor (VEGF) expression vector which when expressed in the cell, can enhance the efficiency of EC isolation and growth. Thus, the use of specific EC mitogens such as VEGF, can shorten the time period from cell harvesting to seeding and thus also allow lower initial graft seeding densities to be used, since the rapid proliferation induced by VEGF will lead to faster graft coverage.

Endothelial cell proliferation and migration is initiated by activation of VEGF receptors (16). Although three VEGF receptors have been identified and characterized, it is the *KDR* receptor that plays a key role in the angiogenic switch that is comprised of proliferation and migration (16,17).

VEGF offers distinct advantages over other less EC-specific growth factors that might enhance graft endothelialization since it has reduced impact on other vascular cells, in particular smooth muscle cells (SMC), and as such reduces the potential for adverse stimulatory effects. Thus, VEGF released by genetically modified cells seeded onto artificial graft will recruit EC, but not SMC, from anastomosis sites.

Although genetically transfected EC over-expressing VEGF are capable of enhanced growth as compared to non-transformed EC, artificial vascular grafts incorporating such transformed cells are functionally limited since the transformed cells coating the luminal surface of the graft are incapable of withstanding flow forces following implantation.

UP50 is a novel protein which, when employed to coat a surface such as a tissue culture plastic, has been found to increase the adherence capacity of EC thereto (51). This protein contains six calcium-binding EGF-like domains, one of which includes an Arg-Gly-Asp (RGD) motif. This motif is known to be involved in the specific binding of integrins, such as those expressed on EC, and thus is involved in mediating adhesion of EC to the cell-matrix and to other cells (52). For example, synthetic peptides containing this RGD sequence are known to antagonize integrin binding and thereby inhibit processes such as angiogenesis or thrombosis (53).

The epidermal growth factor (EGF) like motif is found in diverse extra cellular matrix (ECM) proteins of vascular wall (54) and is involved with calcium binding in several ECM proteins present in vessel walls (55). This motif is also present in regulators of blood coagulation, low density lipoprotein receptor, TGF-β-binding protein and Notch and its ligands, which are involved in cellular differentiation (56).

It has been shown that embryonic vascular EGF-like repeat-containing protein (EVEC), the rat homolog of human UP50, is expressed during embryonic angiogenesis. The expression of this protein during embryogenesis was demonstrated predominantly in the arterial vasculature, mainly in the vascular SMC of the developing arteries. In adult mice the expression of this protein was shown to be down-regulated except in arteries of the uterus. In a model of balloon-injured rat carotid artery EVEC expression was upregulated (57). These finding suggest a possible role for UP50 in EC-SMC interactions, ECM maturation and growth regulation. Thus, UP50 is a novel adherent molecule which may be employed as a vascular ligand mediating adhesion of EC via their integrin receptors and may also promote vascular development and remodeling. However, the strategy of genetically modifying EC to express such adhesive factors capable of promoting endothelialization of synthetic vascular grafts has not been attempted.

There is thus a widely recognized need for and it would be highly advantageous to have an Artificial vascular graft capable of supporting blood flow following implantation and yet devoid of the abovementioned limitations of prior art grafts.

### SUMMARY OF THE INVENTION

According to one aspect of the present invention there is provided an artificial vascular graft comprising a synthetic tubular element having a luminal surface being coated with a plurality of endothelial cells and/or smooth muscle cells being genetically transformed to express at least one cell proliferating growth factor and at least one cellular adherence factor.

According to another aspect of the present invention there is provided a method of replacing or bypassing at least a portion of a vascular system of an individual, the method comprising implanting into the vascular system of the individual an artificial vascular graft, so as to form a fluid communication between the vascular system and the artificial vascular graft, the artificial vascular graft including a synthetic tubular element having a luminal surface being coated with a plurality of endothelial cells and/or smooth muscle cells being genetically transformed to express at least one cell proliferating growth factor and at least one cellular adherence factor.

According to yet another aspect of the present invention there is provided a method of producing an artificial vascular graft, the method comprising: (a) genetically transforming endothelial cells and/or smooth muscle cells to express at least one cell proliferating growth factor and at least one cellular adherence factor; and (b) seeding and culturing the endothelial cells and/or smooth muscle cells within a synthetic tubular element having a luminal surface until sufficient coating of the luminal surface is achieved.

According to further features in preferred embodiments of the invention described below, a first portion of the plurality of endothelial cells and/or smooth muscle cells is genetically transformed to express the at least one cell proliferating growth factor and further wherein a second portion of the plurality of endothelial cells and/or smooth muscle cells is genetically transformed to express the at least one cellular adherence factor.

According to still further features in the described preferred embodiments the luminal surface is of a substance selected from the group consisting of polytetrafluoroethylene (PTFE), expanded polytetrafluoroethylene (ePTFE), polyester fibers, Dacron^{™}, processed animal blood vessels, or any artificial scaffold material used in graft fabrication.

According to still further features in the described preferred embodiments the synthetic tubular element is of an inner cross sectional area which is substantially equivalent to an inner cross sectional area of a blood vessel.

According to still further features in the described preferred embodiments the inner cross sectional area of the synthetic tubular element is within a range of about 7 to 700 mm².

According to still further features in the described preferred embodiments the plurality of endothelial cells are derived from a source selected from the group consisting of a segment of a vein, bone marrow, peripheral blood progenitor cells and circulating endothelial cells.

According to still further features in the described preferred embodiments the plurality of smooth muscle cells are derived from a source selected from the group consisting of human saphenous veins and left internal mammary arteries, bone marrow, and peripheral blood progenitor cells.

According to still further features in the described preferred embodiments the plurality of endothelial cells and/or smooth muscle cells are derived from the recipient of the artificial vascular graft.

According to still further features in the described preferred embodiments the plurality of endothelial cells and/or smooth muscle cells are derived from embryonic or mature tissues of a human or an animal donor.

According to still further features in the described preferred embodiments the plurality of endothelial cells form a confluent monolayer at the inner luminal surface.

According to still further features in the described preferred embodiments at least one cell proliferating growth factor is selected from the group consisting of VEGF, basic or acidic FGF, HGF and any other endothelial growth factor.

According to still further features in the described preferred embodiments at least one cellular adherence factor is DANCE, UP50, vitronectin, albumin, collagen I, collagen IV, fibronectin and laminin or any other molecule capable of promoting cellular adherence to graft material.

According to still further features in the described preferred embodiments the plurality of endothelial cells and/or smooth muscle cells are further genetically transformed to express at least one marker polypeptide.

According to still another aspect of the present invention there is provided a nucleic acid expression construct comprising: (a) a first polynucleotide segment encoding a cell proliferating growth factor; and (b) a second polynucleotide segment encoding cellular adherence factor.

According to still another aspect of the present invention there is provided a nucleic acid expression construct system comprising: (a) a first nucleic acid expression construct including a first polynucleotide segment encoding a cell proliferating growth factor; and (b) a second nucleic acid expression construct including a second polynucleotide segment encoding cellular adherence factor.

According to still further features in the described preferred embodiments the first and the second nucleic acid expression constructs further including at least one additional polynucleotide segment encoding a marker polypeptide.

According to still another aspect of the present invention there is provided a kit comprising a stand for engaging at least one tube, the at least one tube including a nucleic acid expression constructs system, the nucleic acid expression construct system including: (a) a first nucleic acid expression construct including a first polynucleotide encoding a cell proliferating growth factor; and (b) a second nucleic acid expression construct including a second polynucleotide encoding cellular adherence factor.

According to still another aspect of the present invention there is provided a kit comprising a stand for engaging at least one tube, the at least one tube including a nucleic acid expression construct, the nucleic acid expression construct including: (a) a first polynucleotide encoding a cell proliferating growth factor; and (b) a second polynucleotide encoding cellular adherence factor.

According to still another aspect of the present invention there is provided method of producing genetically transformed endothelial cells and/or smooth muscle cells, the method comprising: (a) obtaining endothelial cells and/or smooth muscle cells from a mammalian tissue source; and (b) transforming the endothelial cells to express at least one cell proliferating growth factor and at least one cellular adherence factor concurrently or separately.

According to still further features in the described preferred embodiments the nucleic acid expression construct further comprising at least one promoter sequence being for directing the expression of at least one of the first and the second polynucleotide segments.

According to still further features in the described preferred embodiments the first polynucleotide segment is transcriptionally linked to the second polynucleotide segment whereas the first and the second polynucleotide segment are under the transcriptional control of a single promoter sequence of the at least one promoter sequence.

According to still further features in the described preferred embodiments the nucleic acid construct further comprising a linker sequence being interposed between the first and the second polynucleotide segments.

According to still further features in the described preferred embodiments the linker sequence is selected from the group consisting of IRES and a protease cleavage recognition site.

According to still further features in the described preferred embodiments at least one promoter is functional in eukaryotic cells.

According to still further features in the described preferred embodiments at least one promoter is selected from the group consisting of a constitutive promoter, an inducible promoter and a tissue specific promoter.

According to still further features in the described preferred embodiments nucleic acid expression construct further comprising: (c) a first promoter sequence being for directing the expression of the first polynucleotide segment; and (d) a second promoter sequence being for directing the expression of the second polynucleotide segment.

According to still further features in the described preferred embodiments the first promoter and the second promoter are selected from the group consisting of a constitutive promoter, an inducible promoter and a tissue specific promoter.

According to still further features in the described preferred embodiments the inducible promoters are regulatable by same effector molecule.

According to still further features in the described preferred embodiments the nucleic acid expression construct, further comprising at least one additional polynucleotide segment encoding a marker polypeptide.

According to still further features in the described preferred embodiments the marker polypeptide is selected from the group consisting of a selection polypeptide and a reporter polypeptide.

According to still further features in the described preferred embodiments at least one additional polynucleotide segment is transcriptionally linked to the at least one of the first and the second polynucleotide segments.

According to still further features in the described preferred embodiments at least one additional polynucleotide segment is transcriptionally linked to the at least one of the first and the second polynucleotide segments via linker segment.

According to still further features in the described preferred embodiments the linker sequence is selected from the group consisting of IRES and a protease cleavage recognition site.

According to still further features in the described preferred embodiments at least one additional polynucleotide segment is translationally fused to at least one of the first and the second polynucleotide segments.

According to still another aspect of the present invention there is provided a transformed endothelial cell and/or smooth muscle cell being genetically transformed to express at least one cell proliferating growth factor and at least one cellular adherence factor.

According to still further features in the described preferred embodiments the transformed endothelial cell and/or smooth muscle cell further genetically transformed to express at least one marker polypeptide.

According to still further features in the described preferred embodiments the endothelial cell is derived from a source selected from the group consisting of a segment of a vein, bone marrow, peripheral blood progenitor cells and circulating endothelial cells.

According to still further features in the described preferred embodiments the endothelial cell or smooth muscle cell is derived from a source selected from the group consisting of embryonic or mature tissues of a human donor and an animal source.

According to still further features in the described preferred embodiments the cellular proliferating growth factor is selected from the group consisting of VEGF, acidic and basic FGF, HGF and any other endothelial cell growth factor.

According to still further features in the described preferred embodiments the cellular adherence factor is DANCE, UP50, vitronectin, albumin, collagen I, collagen IV, fibronectin and laminin or any other molecule capable of promoting cellular adherence to graft material.

According to still another aspect of the present invention there is provided an artificial vascular graft comprising a synthetic tubular element having a luminal surface being coated with endothelial cells and smooth muscle cells, at least one of the endothelial cells and smooth muscle cells being genetically transformed to express at least one cell proliferating growth factor and at least one cellular adherence factor.

According to yet another aspect of the present invention there is provided a method of producing an artificial vascular graft, the method comprising: (a) providing endothelial cells and smooth muscle cells, at least one of the endothelial cells and smooth muscle cells being genetically transformed to express UP50 and VEGF; and (b) sequentially or simultaneously culturing the endothelial cells and the smooth muscle cells within a synthetic tubular element having a luminal surface until sufficient coating of the luminal surface with the endothelial cells and smooth muscle cells is achieved.

According to yet another aspect of the present invention there is provided an artificial vascular graft comprising a synthetic tubular element having a luminal surface coated with a plurality of endothelial cells and an exterior surface coated with a plurality of smooth muscle cells.

According to preferred embodiments of the present invention the plurality of endothelial cells are genetically transformed to express at least one cell proliferating growth factor and at least one cellular adherence factor.

According to preferred embodiments of the present invention the plurality of smooth muscle cells are genetically transformed to express at least one cellular adherence factor.

The present invention successfully addresses the shortcomings of the presently known configurations by providing an artificial vascular graft and method of generating same, which graft is useful for vascular replacement or vascular bypass.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a perspective view of an artificial vascular graft of the present invention.
FIG. 2 is a flowchart depicting the strategy for constructing the hybrid biological-synthetic vascular grafts of the present invention.
FIGs. 3a-b are schematics illustrating adenoviral and retroviral constructs for expression of VEGF-GFP (Figures 3a and 3b, respectively).
FIGs. 4a-d are schematics depicting viral expression vectors for expression of Urine p50 protein (UP50). Figures 4a and 4b depict adenoviral vectors for expression of UP50 and UP50-GFP, respectively, and Figures 4c and 4d depict retroviral vectors for expression of UP50 and UP50-GFP, respectively.
FIGs. 5a-b are photomicrographs depicting PTFE (polytetrafluoroethylene) grafts seeded with human EC infected with recombinant adenoviral vectors encoding nuclear targeted LacZ gene (Figure 5a) or both VEGF and green fluorescent protein (GFP) genes (Figure 5b).
FIGs. 6a-b are photomicrographs depicting PTFE (polytetrafluoroethylene) grafts seeded with human EC transduced with recombinant retroviral vectors encoding nuclear targeted LacZ gene (Figure 6a) or both VEGF and green fluorescent protein (GFP) genes (Figure 6b).
FIGs. 7a-d are photomicrographs depicting EC (Figures 7a-b) and SMC (Figure 7b-d) infected with Ad.UP50-GFP. Cells were visualized by either light (Figures 7a and 7c) or fluorescent (Figures 7b and 7d) microscopy.
FIGs. 8a-d are photomicrographs depicting EC (Figures 8a and 8b) and SMC (Figure 8c and 8d) retrovirally transduced to express UP50-GFP. Cells were visualized by light (Figures 8a and 8c) and fluorescent (Figures 8b and 8d) microscopy.

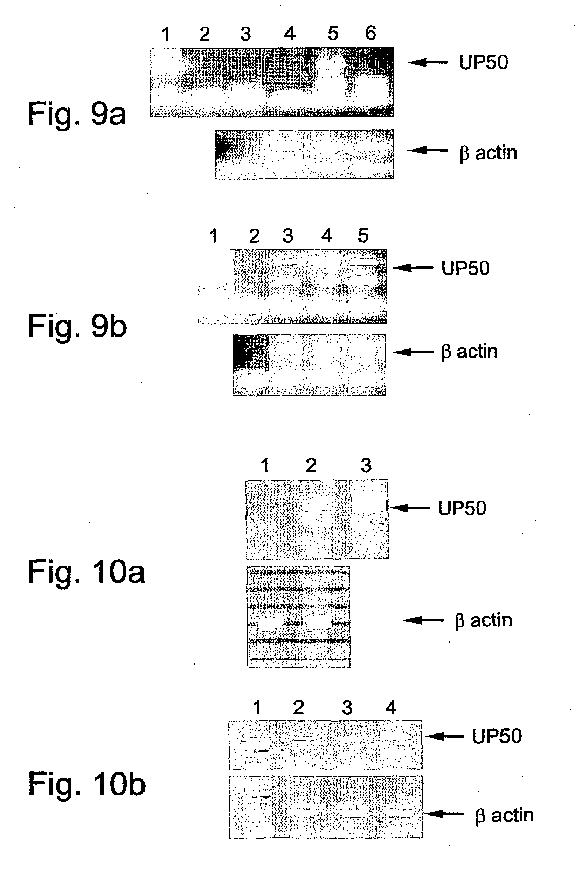

FIGs. 9a-b are photographs depicting RT-PCR analysis of UP50 mRNA expression in EC and SMC adenovirally-infected to express UP50 (Figures 9a and 9b, respectively). Lanes in Figure 9a are as follows: 1. positive control of plasmid UP50 DNA; 2. Negative control of reverse transcriptase reaction; 3. Negative control of PCR reaction; 4. Ad.GFP infected EC; 5. Ad.UP-GFP infected EC; 6. Non infected EC. Lanes in Figure 9b are as follows: 1. positive control plasmid UP50 cDNA; 2. Negative control of reverse transcriptase reaction; 3. Ad.GFP infected SMC; 4. Ad.UP50-GFP infected SMC; 5. Non infected SMC.
FIGs. 10a-b are photographs depicting RT-PCR analysis of UP50 mRNA expression in retrovirally transduced EC and SMC (Figures 10a and 10b, respectively). Lanes in Figures 10a are as follows: 1. Retro-GFP transduced EC, 2. EC retrovirally infected to express UP50 3. positive control of plasmid UP50 DNA. Lanes in Figure 10b are as follows: 1.Marker, 2. non-transduced SMC; 3. retroGFP transduced SMC; 4. retro UP50-GFP transduced SMC.
FIG. 11 is a photograph depicting Western immunoblot analysis of UP50 protein expression by EC and SMC adenovirally-infected to express UP50. Lanes are as follows: 1-Control non-infected EC, 2-Ad.GFP-infected EC, 3-Ad.UP50-GFP infected EC, 4-Ad.Ang1-GFP infected EC, 5-Control non-infected SMC, 6-Ad.GFP infected SMC, 7-Ad.UP50-GFP infected SMC, 8-Ad.Ang1-GFP infected SMC, 9- positive control of 293-FLYA stable transformant expressing UP50-GFP.
FIG. 12 is a photograph depicting Western immunoblot analysis of UP50 protein expression by retrovirally-transduced EC and SMC. Lanes are as follows: 1-Non-transduced EC, 2- retroGFP transduced EC, 3-retroUP50-GFP transduced EC, 4-Non-transduced SMC, 5-retroGFP transduced SMC, 6- retroUP50-GFP transduced SMC, 7- positive control of 293-FLYA stable transformant expressing UP50-GFP.
FIGs. 13a-d are fluorescence photomicrographs depicting immunohistochemical analysis of UP50 surface expression in EC (Figures 13a and 13b) and SMC (Figures 13c and 13d) infected with Ad.UP50-GFP (Figures 13b and 13d) or with control plasmid (Figures 13a and 13a).
FIGs. 14a-c are fluorescent scanning confocal photomicrographs depicting EC infected with Ad.UP50 (Figure 14a) or Ad.UP50-GFP (Figures 14b and 14c). Figures 14b and 14c depict staining of UP50 in EC growing with or without cell-cell contact, respectively. Green and red fluorescence represent staining of GFP and UP50, respectively.
FIG. 15 is a fluorescence photomicrograph depicting immunohistochemical analysis of UP50 protein in ECM generated by Ad.UP50-GFP infected EC.
FIGs. 16a-b depict Western immunoblotting analysis of VEGF and UP50 expression (Figures 16a and 16b, respectively) by co-cultures of adenovirally infected EC and SMC. Lanes are as follows: 1-Ad.VEGF-GFP infected EC + Ad.UP50-GFP infected EC, 2-Ad.Ang1-GFP infected EC + Ad.UP50-GFP infected EC, 3-Ad.VEGF-GFP infected EC + Ad.UP50-GFP infected SMC, 4-Ad.VEGF-GFP infected EC + Ad.Ang1-GFP infected SMC, 5- positive control of 293-FLYA stable tranfectant expressing UP50.

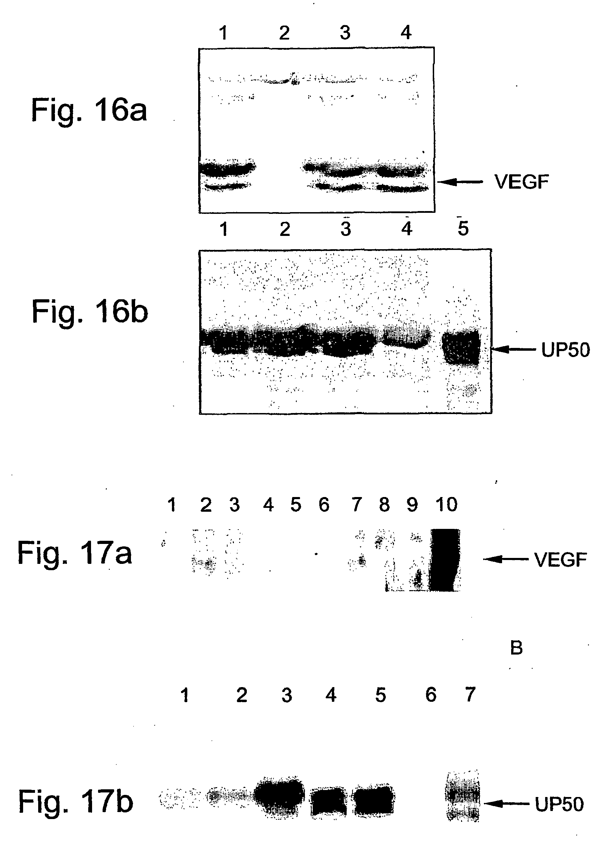

FIGs. 17a-b depict Western immunoblotting analysis of VEGF and UP50 expression (Figures 17a and 17b, respectively) by co-cultures of retrovirally-infected EC and SMC. Lanes are as follows. Figure 17a: 1) retroUP50-GFP transduced EC + retroGFP transduced EC; 2) retroVEGF-GFP transduced EC + retroUP50-GFP transduced EC; 3) retro VEGF-GFP transduced EC, 4) retroUP50-GFP transduced EC, 5) retroGFP transduced EC, 6)EC control, 7) retroUP50-GFP transduced SMC + retroVEGF-GFP transduced EC, 8) retroUP50-GFP transduced SMC + retroGFP transduced EC, 9) SMC control, 10) recombinant VEGF positive control. Figure 17b: 1) SMC control, 2) retroGFP transduced SMC, 3) retroUP50-GFP transduced SMC + retroVEGF-GFP transduced EC, 4) retroUP50-GFP transduced SMC + retroGFP transduced EC, 5) retroUP50-GFP transduced SMC, 6) VEGF control, 7) UP50 control.
FIG. 18 is a histogram depicting proliferation of EC infected with adenoviral vectors encoding UP50-GFP, VEGF-GFP or GFP.
FIGs. 19a-c are fluorescence photomicrographs depicting GFP expression in retroGFP transduced EC (Figure 19a) and EC transduced with retroUP50-GFP and subsequently infected with either Ad.GFP or Ad.VEGF-GFP (Figures 19b and 19c, respectively).
FIGs. 20a-b are photographs depicting Western immunoblotting analysis of VEGF and UP50 protein expression (Figures 20a and 20b, respectively) in retroUP50-GFP transduced EC subsequently infected with adenoviral vectors. Lanes are as follows: 1- positive control of 293-FLYA stable expressing UP50, 2-control non-infected EC, 3-Ad.GFP infected EC, 4-Ad.VEGF-GFP infected EC, 5- EC transduced with retroUP50-GFP, 6- EC transduced with retro-UP50-GFP and infected with Ad.GFP, 7- EC transduced with retroUP50-GFP and infected with Ad.VEGF-GFP, C-positive control of recombinant VEGF.
FIGs. 21a-h are light (Figures 21a, 21c, 21e and 21g) or fluorescence (Figures 21b, 21d, 21f and 21h) photomicrographs depicting 3-dimensional *in vitro* angiogenesis of Ad.GFP (Figures 21a-d) or Ad.UP50-GFP (Figures 21e-h) infected EC without (Figures 21a, 21b, 21e and 21f) or with (Figures 21c, 21d, 21g and 21h) addition of 100 ng/ml VEGF. Representative assays of each experimental group are shown.
FIGs. 22a-h are light (Figures 22a, 22c, 22e and 22g) or fluorescence (Figures 22b, 22d, 22f and 22h) photomicrographs depicting 3-dimensional *in vitro* angiogenesis of Ad.GFP (Figures 22a-d) or Ad.UP50-GFP + Ad.GFP (Figures 22e-h) infected EC without (Figures 22a, 22b, 22e and 22f) or with (Figures 22c, 22d, 22g and 22h) 50% co-culture with Ad.VEGF-GFP infected EC. Representative assays of each experimental group are shown.
FIG. 23 is a histogram depicting an adhesion assay of Ad.UP50 (burgundy bars), Ad.GFP (blue bars) or non- (yellow bars) infected EC. Two different experiments, performed in triplicates are presented.
FIG. 24 is a histogram depicting adhesion of EC to UP50-containing ECM. Results are presented as corrected OD values after subtraction of background.

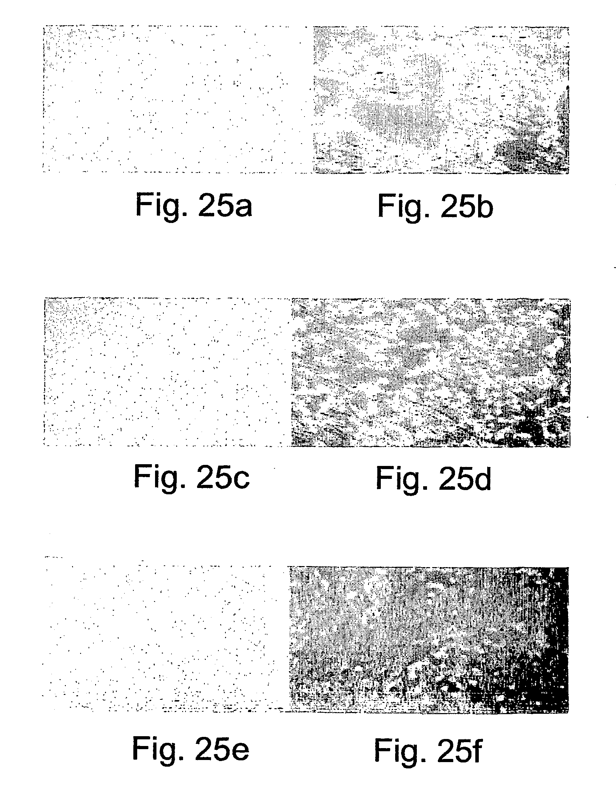

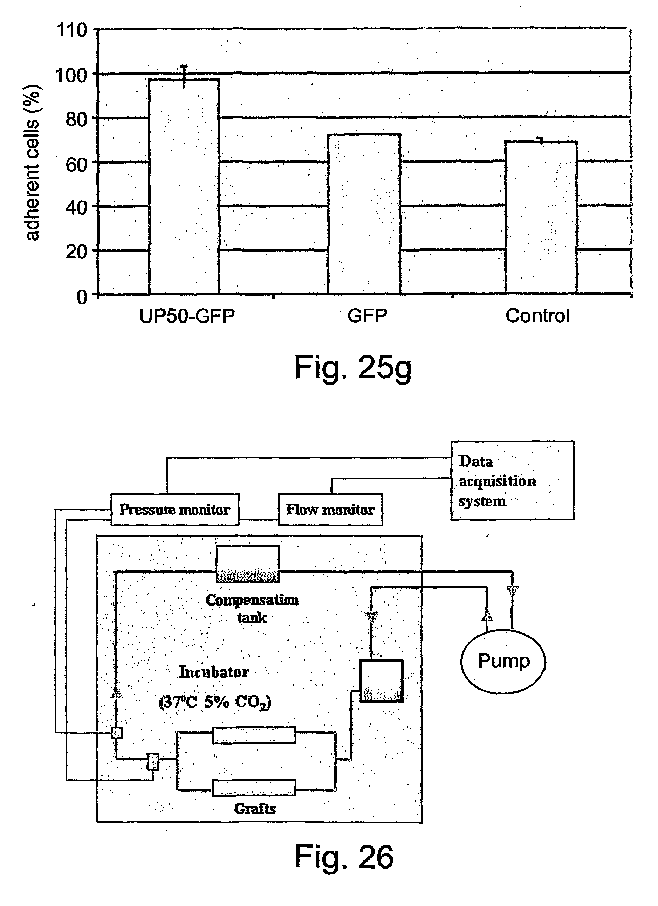

FIGs. 25a-g depict adhesion of UP50-overexpressing EC exposed to continuous shear stress. Randomly selected fields depicting control non-(Figures 25a and 25b), retroGFP- (Figures 25c and 25d) and retroUP50-(Figures 25e and 25f) infected EC are depicted prior to (Figures 25a, 25c and 25e) and following (Figures 25b, 25d and 25f) 24 hours of rocking. Figure 25h is a histogram depicting a count of the number of cells remaining after 24 hours of rocking. Prior to the experiment 2 wells from each group (UP50-GFP, GFP, Control) were counted. Results are shown as percent remaining cells number of the total number counted prior to start of rocking.
FIG. 26 is a schematic depicting the system employed in the present invention for testing cell retention under laminar flow conditions in vitro.
FIGs. 27a-d depict the various components of the system employed in the present invention for testing cell retention under laminar flow conditions in vitro. Figure 27a depicts the tissue culture incubator containing the flow system, Figure 27b depicts the pressure and flow monitors, Figure 27c depicts the data acquisition system and pump and Figure 27d depicts the endothelial seeding device.
FIGs. 28a-d are fluorescence photomicrographs depicting ePTFE synthetic vascular grafts seeded with EC retrovirally transduced to express GFP (Figures 28a and 28b) or UP50-GFP (Figures 28c and 28d) exposed to pulsatile laminar flow conditions in a laminar flow system.
FIG. 29 is an photograph depicting an exposed sheep femoral artery bearing an implanted ePTFE synthetic vascular graft seeded with genetically modified human EC.
FIGs. 30a-b are angiograms depicting the surgical field of PTFE-graft arterial anastomosis and patency of implanted grafts in the left and right femoral arteries (Figures 30a and 30b, respectively).
FIGs. 31a-f are fluorescence photomicrographs depicting cell retention on the luminal surfaces of *in vivo*-implanted grafts seeded with EC retrovirally-transduced to express GFP (Figures 31a and 31b), VEGF-GFP (Figures 31c and 31d) or UP50-GFP (Figures 31e and 31f). Two representative fields are shown for each assay condition.

### DESCRIPTION OF THE PREFERRED EMBODIMENTS

The present invention is of an artificial vascular graft which can be utilized to replace or bypass damaged and/or occluded blood vessels and of methods of generating and transplanting same. Specifically, the present invention is of a method of generating synthetic vascular grafts coated with mammalian endothelial cells which grafts can provide a viable alternative to presently utilized natural and synthetic vascular grafts.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

Artificial grafts are yet to be extensively utilized in surgical bypass procedures. One of the limitations plaguing presently available artificial vascular grafts as well as some natural vascular grafts is the lack of durability over extended periods of time. Although the use of endothelial cells greatly enhances the quality of coated artificial grafts, by improving performance in general and by improving thrombolytic activity in particular, such grafts typically suffer from loss of cell coating under blood flow forces since the coated cells cannot withstand the shear forces exerted thereupon by flowing blood.

Various attempts have been made to improve graft durability. U.S. Patent No. 5,785,965 describes an artificial graft seeded with endothelial cells genetically transformed to express VEGF. Although such cells exhibit improved graft seeding properties, as demonstrated in the Examples section which follows, grafts seeded with endothelial cells expressing VEGF cannot durably withstand forces exerted thereupon by flowing blood.

While experimenting with graft seeding approaches, the present inventors have uncovered that a genetically transformed population of endothelial cells and/or smooth muscle cells which co-express a cellular adherence factor, such as UP50 along with a cellular growth factor, such as VEGF, or a mixture of two subpopulations of genetically modified endothelial cells and/or smooth muscle cells, one expressing a cellular adherence factor and the other a cell growth factor are advantageous for use in seeding artificial vascular grafts.

As is evident from the results presented the Examples section which follows, expression of a cellular adherence factor in graft seeded cells does not retard cell proliferation while it greatly increases the adherence of such cells to the luminal surface of the artificial graft substrate and as such, greatly increases the durability of the vascular graft. In addition, as is clearly demonstrated in the Examples section, co-expression of the cellular adherence factor and the cell proliferation factor leads to complete coating of the graft luminal surface with endothelial cells which are capable of withstanding the forces exerted by the flow of blood therein.

In sharp contrast to prior art vascular grafts which are pretreated to include a cellular adherence factor such as, fibronectin (35-37 and 39), expression of a cellular adherence factor such as, UP50, from the graft-seeded cells along with expression of a cell proliferating factor such as VEGF from these or other seeded cells, substantially improves cell-to-cell and cell-to-graft adherence and as such substantially improves graft durability.

In addition, since the cellular adherence factor(s) expressed by the seeded cells of the present invention form a part of the extracellular matrix which directs cell-to-cell and cell-to graft interactions, release of such factors into circulation following graft implantation is minimized. In sharp contrast, incorporation of such factors in vascular graft material, as is effected by prior art vascular grafts, can lead to release of such factors into circulation following implantation and rapid dilution of this factors, thus leading to loss of activity.

Such release of graft mobilized adherence factors can also lead to thrombosis since such factors can activate the coagulation system and/or cause local inflammation, processes which substantially shorten graft survival and present a severe health risk to the patient.

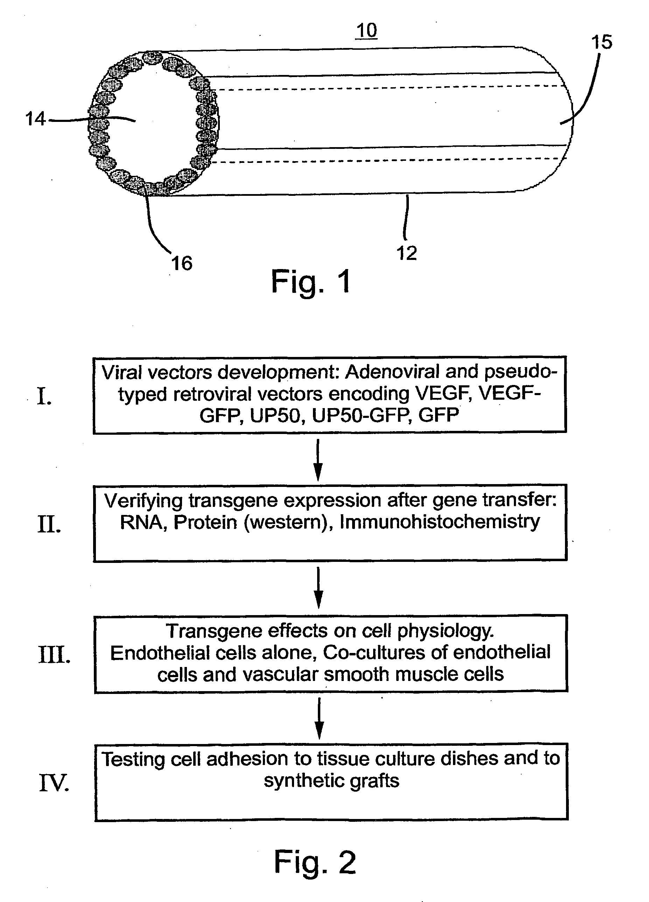

Thus according to one aspect of the present invention and as specifically shown in Figure 1, there is provided an artificial vascular graft which is referred to hereinunder as graft **10**.

Graft **10** includes a synthetic tubular element **12** having a luminal surface **14**. Preferably, synthetic tubular element **12** is of an inner cross sectional area of about 7 to 700 mm² or any cross sectional area or diameter which is substantially equivalent to an inner cross sectional area or diameter of a blood vessel.

Luminal surface **14** is fabricated from a substance including but are not limited to, polytetrafluoroethylene (PTFE), expanded polytetrafluoroethylene (ePTFE), polyester fibers, Dacron^{™} or processed blood vessels derived from an animal or a human.

Luminal surface **14** preferably includes structures such as pits and/or projections, or any other structures which can facilitate/improve cell seeding thereupon.

As is further described hereinunder and in the Examples section which follows, luminal surface **14** is coated with a plurality of cells **16** including endothelial cells and/or smooth muscle cells, at least a portion of which are genetically transformed to express at least one cell proliferating growth factor and at least one cellular adherence factor.

Alternatively, luminal surface **14** can be coated with endothelial cells and optionally smooth muscle cells each being genetically transformed to express at least one cell proliferating growth factor and at least one cellular adherence factor, while an external surface **15** of tubular element **12** can be coated with transformed or non-transformed smooth muscle cells. Such a configuration can substantially improve graft durability and acceptance.

According to preferred embodiments of the present invention, the cell proliferating growth factor is, for example, VEGF (GenBank Accession number AB021221), acidic or basic FGF (GenBank Accession numbers S67291 and M27968 respectively) or HGF (GenBank Accession number D14012), while the cellular adherence factor is, for example, UP50 (SEQ ID NO:1), DANCE (GenBank Accession number AF112152), vitronectin (GenBank Accession number NM000638), albumin (GenBank Accession number NM000477), collagen I (GenBank Accession number J00114), collagen IV (GenBank Accession number M15524 ), fibronectin (GenBank Accession number X02761) or laminin (GenBank Accession number NM005560) or any other molecule capable of promoting endothelial/smooth muscle cell adherence to the graft material. References 34-45 of the References section hereinunder provide evidence supporting the applicability of the abovementioned proliferating growth factors and cellular adherence factors in graft **10** of the present invention.

Preferably, when both endothelial and smooth muscle cells are utilized, the cell proliferating growth factor is specific to endothelial cells such that unwanted proliferation of smooth muscle cells is avoided.

To fabricate graft **10** of the present invention, cells **16** are seeded/cultured within element **12** until a sufficient degree of coating of luminal surface **14** is achieved. Preferably, cells **16** are cultured under conditions conducive for the formation of a confluent monolayer upon luminal surface **14**.

Several seeding approaches can be used to coat luminal surface **14** of graft **10** of the present invention.

In cases where both endothelial cells and smooth muscle cells are utilized, sequential seeding of luminal surface **14** is preferred. In such a case, transformed or non-transformed smooth muscle cells are preferably seeded first followed by seeding with transformed endothelial cells.

Alternatively, graft **10** can be seeded with transformed smooth muscle cells and subsequently implanted within the body thus enabling circulating endothelial cells to coat (endothelialize) luminal surface **14**.

It will be appreciated that cells **16** can be transformed with both the cell proliferating growth factor and the cellular adherence factor, or alternatively a first portion of such cells can be transformed with the cell proliferating growth factor, while a second portion of these cells is transformed with cellular adherence factor.

The endothelial cells and smooth muscle cells used by the present invention can be derived from various mammalian tissue sources.

According to a preferred embodiment of the present invention, endothelial cells are prepared from, for example, a segment of a vein, bone marrow progenitor cells, peripheral blood stem cells or circulating endothelial cells.

According to another preferred embodiment of the present invention, smooth muscle cells are prepared from, for example, human saphenous veins, left internal mammary arteries, bone marrow progenitor cells, and peripheral blood stem cells.

In any case, cells used by the present invention are typically recovered from tissues of the intended recipient of graft **10** or a syngeneic donor.

Alternatively xenogeneic tissue can also be utilized for the preparation of such cells providing measures are taken prior to, or following fabrication of graft **10**, so as to avoid cell rejection. Numerous methods for preventing or alleviating rejection are known in the art and as such no further detail is given herein.

As is further described in the Examples section below, co-expression of a cellular adherence factor along with a cellular growth factor enables cells **16** of graft **10** of the present invention to withstand flow forces which would normally lead to cell loss from the luminal surface of prior art artificial vascular grafts.

Thus, unlike prior art synthetic grafts, graft **10** of the present invention can be successfully utilized in vascular surgical procedures aimed at replacing or bypassing a damaged or occluded blood vessel. In such procedures graft **10** is implanted into the vascular system of the individual so as to form a fluid communication between the vascular system and graft **10.**

As mentioned hereinabove, the transformed endothelial and/or smooth muscle cells according to the teachings of the present invention express at least one cell proliferating growth factor and at least one cellular adherence factor.

Such factors can be encoded by sequences derived from human cells or any other mammalian cells provided the expressed factors are functional in endothelial/smooth muscle cells.

Such factors can be expressed from exogenous polynucleotide sequences introduced into the cells, or alternatively such factors can be endogenous factors naturally expressed by the cell in which case, the cell is transformed with sequences directed at causing the overexpression of the endogenous factors.

As used herein, the term "overexpression" refers to expression levels which exceeded those normally found in a cell. Over expression can be effected by introducing additional copies of an endogenous gene into a cell thus increasing the copy number of the endogenous gene or genes expressed in a cell, or by introducing enhancer sequence elements via gene knock-in procedures, which elements upregulate the transcription or translation of the endogenous genes.

The exogenous polynucleotide sequences are preferably included in one or more nucleic acid constructs which are used to genetically transform the cells.

As used herein the phrase "genetically transform" refers to the introduction of exogenous polynucleotide sequences into a cell. Such sequences can integrate into the genome of the cell, or alternatively, such exogenous sequences can exist in the nucleus or cytoplasm of the cell in a transient manner.

Thus, according to another aspect of the present invention there is provided a nucleic acid expression construct useful for generating cells **16** of the present invention. The construct includes a first polynucleotide segment encoding a cell proliferating growth factor and a second polynucleotide segment encoding a cellular adherence factor.

The construct according to this aspect of the present invention also includes one or more promoter sequences for directing the expression of the first and second polynucleotide segments in the transformed cells. Such a promoter can be any mammalian functional promoter which is either constitutive, tissue specific or inducible.

As is further described in the Examples section herein below, the cell proliferating growth factor and the cellular adherence factor are preferably expressed at a different temporal pattern.

As such the construct includes two promoter sequences each being for directing the expression of a single polynucleotide segment. Preferably such promoters are inducible promoters regulatable by the same effector molecule. Preferably, the promoter sequences are selected such that one promoter is upregulatable by an effector molecule, while the second promoter is downregulatable by the same effector.

Examples of suitable inducible promoters include, but are not limited to chemically (effector molecule) induced promoters, such as the promoters utilized by the Tet-On (TM) and Tet-Off (TM) gene expression systems commercially available from Clontech, or shear stress induced promoters such as those described in [46-50].

Not withstanding from the above, it will be appreciated that a single promoter sequence can also be utilized by the construct of the present invention provided the first and second polynucleotide segments are transcriptionally linked and provided that an internal ribosome entry site (IRES) is included for directing the translation of the second segment of the polycistronic message transcribed.

The two polynucleotide segments of the construct according to this aspect of the present invention can also be translationally fused provided a protease cleavage site is provided therebetween which allows cleavage and separation of the two polypeptides in expressing cells.

It will further be appreciated that each of the two polynucleotide segments can be included in a separate nucleic acid constructs, which can be co-introduced into the cells.

Thus according to yet another aspect of the present invention there is provided a nucleic acid expression construct system useful in generating cells **16** of the present invention. The construct system of this aspect of the present invention includes a first nucleic acid expression construct including a first polynucleotide segment encoding a cell proliferating growth factor and a second nucleic acid expression construct including a second polynucleotide segment encoding cellular adherence factor.

The construct or construct system of the present invention preferably also includes additional polynucleotides segments encoding marker genes, selection marker genes and the like, so as to enable selection of transformed cells and/or monitoring of the expression of the cell proliferating growth factor and/or the cellular adherence factor. Examples of suitable reporter genes include, but are not limited to, beta-galactosidase, luciferase, GFP and the like. Examples of selection markers include antibiotic resistance genes and the like.

It will be appreciated that to monitor expression of the cell proliferating growth factor and/or the cellular adherence factor, the reporter gene can be provided in transcriptional or translational fusion to the polynucleotide segment encoding the factor or it can be placed under a transcriptional control of a promoter sequence identical to that directing the transcription of the factor.

It will be appreciated that the polynucleotide segments encoding the cell proliferating growth factor and/or the cellular adherence factor, can be ligated into a commercially available expression vector system suitable for transforming mammalia cells and for directing the expression of these factors within the transformed cells. It will be appreciated that such commercially available vector systems can easily be modified via commonly used recombinant techniques in order to replace, duplicate or mutate existing promoter or enhancer sequences and/or introduce any additional polynucleotide sequences.

Suitable mammalian expression vectors include, but are not limited to, pcDNA3, pcDNA3.1(+/-), pZeoSV2(+/-), pSecTag2, pDisplay, pEF/myc/cyto, pCMV/myc/cyto, pCR3.1, which are available from Invitrogen, pCI which is available from Promega, pBK-RSV and pBK-CMV which are available from Stratagene, pTRES which is available from Clontech, and their derivatives.

As mentioned hereinabove, the transformed cells of the present invention can also include exogenous polynucleotide sequences directed at over expressing endogenously encoded factors.

Thus, according to yet another aspect of the present invention, there is provided a nucleic acid construct or construct system which serves to upregulate expression of endogenously encoded factors. Such a construct or constructs can include transcriptional regulatory sequences which when provided in cis to endogenous sequences encoding the cell proliferating growth factor and/or the cellular adherence factor, can upregulate the transcription thereof.

Alternatively, such a construct or constructs can encode translational regulatory sequences which when provided in trans to endogenous sequences encoding the cell proliferating growth factor and/or the cellular adherence factor, can upregulate the translation thereof.

Standard gene knock-in techniques can be utilized to introduce cis acting transcriptional regulatory sequences into a genome of the endothelial or smooth muscle cells. For a review of gene knock-in methodology see, for example, United States Patent Nos. 5,487,992, 5,464,764, 5,387,742, 5,360,735, 5,347,075, 5,298,422, 5,288,846, 5,221,778, 5,175,385, 5,175,384, 5,175,383, 4,736,866 as well as Burke and Olson, Methods in Enzymology, 194:251-270, 1991; Capecchi, Science 244:1288-1292, 1989; Davies et al., Nucleic Acids Research, 20 (11) 2693-2698, 1992; Dickinson et al., Human Molecular Genetics, 2(8):1299-1302, 1993; Duff and Lincoln, "Insertion of a pathogenic mutation into a yeast artificial chromosome containing the human APP gene and expression in ES cells", Research Advances in Alzheimer's Disease and Related Disorders, 1995; Huxley et al., Genomics, 9:742-750 1991; Jakobovits et al., Nature, 362:255-261 1993; Lamb et al., Nature Genetics, 5: 22-29, 1993; Pearson and Choi, Proc. Natl. Acad. Sci. USA, 1993, 90:10578-82; Rothstein, Methods in Enzymology, 194:281-301, 1991; Schedl et al., Nature, 362: 258-261, 1993; Strauss et al., Science, 259:1904-1907, 1993, WO 94/23049, WO93/14200, WO 94/06908 and WO 94/28123 also provide information.

The nucleic acid constructs of the present invention can be introduced into the endothelial/smooth muscle cells via any standard mammalian transformation method. Such methods include, but are not limited to, direct DNA uptake techniques, and virus or liposome mediated transformation (for further detail see, for example, "Methods in Enzymology" Vol. 1-317, Academic Press).

Thus, the present invention provides an improved artificial vascular graft which is internally coated with genetically transformed cells including endothelial and/or smooth muscle cells expressing a cellular growth factor and a cellular adhesion factor. Expression of the cellular adherence factor, in addition to the cellular growth factor, enables the transformed cells to adhere with greater force to the luminal surface of the artificial vascular graft thereby providing a durable cellular coating which can withstand, for extended periods of time, the flow forces exerted upon the luminal surface of the artificial vascular graft following implantation.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J.E., ed. (1994); Stites et al. (eds), "Basic and Clinical Immunology" (8th Edition); Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents, patent applications and sequences identified by their accession numbers mentioned in this specification are herein incorporated in their entirety by reference into the specification, to the same extent as if each individual publication, patent, patent application or sequence identified by their accession number was specifically and individually indicated to be incorporated herein by reference. In addition, citation or identification of any reference in this application shall not be construed as an admission that such reference is available as prior art to the present invention.

### EXAMPLE 1

### Strategy for generation of vascular grafts

In order to demonstrate the advantages of the improved synthetic vascular grafts (Figure 1) of the present invention it was necessary to formulate a genetic transformation strategy for vascular cells and to design experiments aimed at proving the feasibility of the approach disclosed therein. Therefore, the flow chart shown in Figure 2 describes the experiments performed to demonstrate the capacity of the methods of the present invention to generate vascular grafts possessing enhanced durability.

### EXAMPLE 2

### Construction of viral vectors for genetic modification of vascular cells

Two types of viral vectors were employed for gene transfer into vascular cells; the first is a highly efficient recombinant adenoviral system leading to high-level transgene expression. Recombinant adenoviral preparations have the advantage of being less complex than preparations of other vectors such as adeno-associated vectors (AAV) and lentiviral-based vectors (21, 22) and, unlike other viral vector systems, adenoviral vectors may be employed subsequent to cellular seeding of grafts as cell division is not essential for transgene expression. The second vector employed was a retroviral vector pseudotyped with GALV glycoprotein. Such pseudotyped vectors have a high affinity for human and sheep EC and SMC (23) and, unlike adenoviral vectors, transduction with retroviral vectors leads to stable transgene expression and transmission of gene expression in daughter cells.

Hence, viral vectors were prepared for the production of genetically modified vascular cells overexpressing pro-endothelialization factors to be employed for seeding vascular grafts, as described below.

### Materials and Methods:

***Generation of recombinant adenoviral vectors encoding the LacZ gene:*** The recombinant adenoviral vectors for expression of bacterial nuclear-targeted β-galactosidase gene were constructed in several steps, consisting of routine prokaryotic cloning and a homologous recombination procedure in the 293-cell line. A 3700bp HindIII-BamHI fragment containing the bacterial β-galactosidase gene (LacZ) (Clontech, CA, USA) was inserted into plasmid pCA3 for constitutive expression under the control of the CMV immediate-early promoter. The resultant plasmid was co-transfected with plasmid pJM17 into 293 cells which constitutively express the adenoviral E1 gene. Plasmid pJM17 contains the adenovirus genome, excluding the E3 region, and including an insert (pBRX) in the E1 region of the virus. Homologous recombination between pCA3 encoding β-galactosidase and pJM17 following transfection replaced the E1 region of pJM17 with the CMV-β-galactosidase expression cassette from pCA3. Plaque formation occurred 2 to 4 weeks following co-transfection after which individual plaques were isolated and viral extracts were amplified therefrom by infection of 293 cells. The titer of each viral stock was determined by plaque assay in 293 cells and viral titers of ~10¹⁰ pfu/ml were obtained. Expression of β-galactosidase by transfectants was confirmed by X-gal staining.

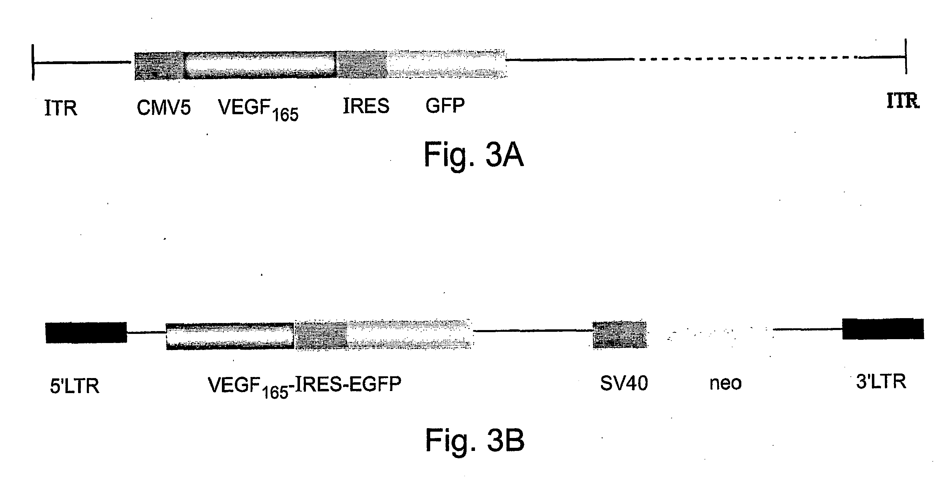

***Generation of recombinant bicistronic adenoviral vectors encoding*** ***the VEGF and GFP genes:*** Recombinant adenoviral vectors encoding the VEGF₁₆₅ and GFP genes were constructed by routine prokaryotic cloning into shuttle vectors followed by homologous recombination in 293 cells. A 600 bp BamHI fragment containing the human VEGF₁₆₅ cDNA (Genbank Accession number AB021221), including the signal sequence for secretion (a kind gift of Dr. J. Abraham, Scios Nova, Mountain View, CA) was inserted into the BgIII site of shuttle vector pQBI-CMV5-GFP (QBI, Canada), thereby generating shuttle vector CMV5-VEGF₁₆₅-IRES-EGFP (Figure 3a). The expression plasmid pQBI-CMV5-GFP contains the left arm (16%) of the Ad5 genome with a deletion in the E1 region containing a hCMV5 insert. Shuttle vector CMV5-VEGF₁₆₅-IRES-EGFP was co-transfected with plasmid pJM17 into 293 cells constitutively expressing the E1 gene. The pJM17 plasmid contains the adenovirus genome, excluding the E3 region, and including an insert (pBRX) in the E1 region of the virus. Homologous recombination between CMV5-IRES-VEGF₁₆₅-GFP and pJM17 following transfection replaced the E1 region and pBRX insert with the expression cassette from CMV5-IRES-VEGF₁₆₅-GFP. Plaque formation occurred 2 to 4 weeks following co-transfection after which individual plaques were isolated and viral extracts were amplified by infection of 293 cells. The titer of each viral stock was determined by plaque assay in 293 cells and titers of ~10¹⁰ pfu/ml were obtained. Transgene expression was confirmed by Western immunoblotting analysis of infected cell-conditioned medium.

***Generation of pseudotyped retroviral vectors encoding human VEGF and GFP:*** Recombinant retroviral vectors encoding the GFP and human VEGF₁₆₅ genes were constructed by cloning into plasmid pLXSN (# K1060-B Clontech, USA) in two steps. In the first step, a 600 bp BamHI fragment encoding VEGF₁₆₅ (Genbank Accession number AB021221) was inserted into the BamHI site of plasmid pIRES2-EGFP (#6029-1 Clontech). Secondly, a 2.0 kB EcoRI-MunI fragment containing the VEGF₁₆₅, IRES and EGFP encoding sequences was cloned into the EcoRI restriction site in pLXSN resulting in vector LXSN-VEGF₁₆₅-IRES-EGFP (Figure 3b). For retroviral vector production, 293E3 ecotropic packaging cells were transiently transfected with LXSN-VEGF₁₆₅-EGFP. After 48 hours supernatant from confluent cultures of G418-resistant producer cells was collected, filtered (0.45 µm) and used to transduce PA317 amphotropic packaging cells. Transduced PA317 cells were grown under G418 selection (300 mg/ml) and after 48 hours supernatant was collected and used to transduce TEFLYGA packaging cells which express GALV envelope glycoprotein to generate pseudotyped virus capable of transducing EC and SMC with high efficiency. After G418 selection (400 µg/ml) of transduced TEFLYGA cells, individual colonies were collected and screened for EGFP and VEGF₁₆₅ expression. Viral titers of each colony were determined by TE671 cell transduction and were found to range from 10⁵ to 10⁶ pfu/ml. The highest-titer producing colonies were selected and freshly collected supernatants were employed for transduction.

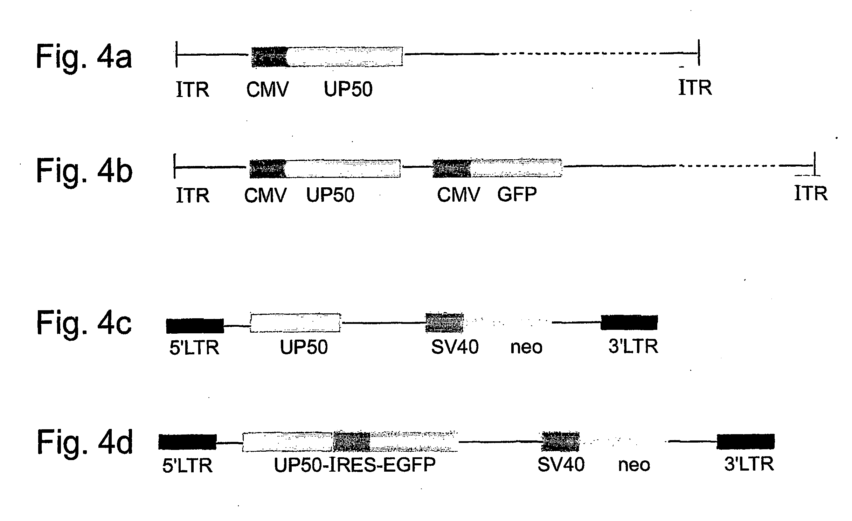

***Generation of recombinant adenoviral vectors encoding the UP50*** ***gene:*** Recombinant adenoviral vector expressing the human UP50 gene (obtained from Y. Shaul, Weizmann Institute) was constructed as described above. A 1361 bp BglII fragment containing the human UP50 cDNA (SEQ ID NO:1) was inserted into plasmid pCA3. Transgene-containing plasmid pCA3 was co-transfected with plasmid pJM17 into 293 cells. Homologous recombination between the expression plasmid and pJM17 following transfection replaced the E1 region with the expression cassette from the pCA3 plasmid thereby generating shuttle vector CMV5-UP50 (Figure 4a). Plaque formation occurred 2-4 weeks following co-transfection. Individual plaques were isolated and viral extracts were amplified by infection of 293 cells. Titers of viral stock of ~10¹¹ pfu/ml were obtained. Transgene expression was confirmed by Western immunoblotting analysis of infected cell-conditioned medium.

***Generation of recombinant adenoviral vectors encoding both the*** ***UP50 and GFP genes:*** A recombinant adenoviral vector co-expressing human UP50 and GFP genes was constructed via a modified AdEasy protocol (28). A 1361 bp BglII fragment of UP50 cDNA was inserted into the BglII site pAdTrack-CMV shuttle vector under the control of the CMV promoter. The shuttle vector encodes GFP under the control of an additional CMV promoter downstream to the transgene. Insert-containing shuttle vector was linearized by PmeI digestion and purified by Qiaquick gel extraction kit (Qiagen, Germany). Competent BJ5183 cells were co-transfected with insert-containing shuttle vector and pAdEasy-1 by electroporation and positive clones containing recombinant adenoviral vector encoding UP50-GFP (Figure 4b) were selected by PCR and restriction map analysis. Recombinant adenoviral plasmids were linearized by PacI digestion, purified and transfected into 293 cells using Lipofectamine 2000 (Gibco BRL, USA). Seven days following transfection, cytopathic effect occurred and 100% of the cells were found to express GFP. The cells were harvested and viral extracts were further amplified in 293 cells. The titer of each viral stock was determined by serial dilution assay in 293 cells and the titers of ~10¹¹ pfu/ml were obtained. Expression of transgene was confirmed by Western immunoblotting analysis of infected cell-conditioned medium.

***Construction of retroviral vectors for expression of UP50 or co-expression of UP50 and EGFP:*** Recombinant retroviral vector LXSN-UP50 encoding the human UP50 gene (Figure 4c) was constructed by inserting the human UP50 cDNA 1361 bp BglII fragment into the BamHI site of plasmid pLXSN (# K1060-B Clontech, USA) under the control of Mo-MULV 5' long terminal repeat (LTR).

A bicistronic recombinant retroviral vector encoding both the UP50 and EGFP genes was cloned into plasmid pLXSN in two steps; first, a 1400 bp IRES-EGFP EcoRI-HpaI fragment excised from pIRES2-EGFP (Clontech, #6029-1) was inserted into EcoRI-Hpal-digested pLXSN for construction of the control plasmid pLXSN-IRES-EGFP. The second step consisted of the construction of pLXSN-UP50-IRES-EGFP (Figure 4d) by cloning of human UP50 EcoRI fragment (1361 bp) into the EcoRI site of pLXSN-IRES-EGFP. Gene expression in these constructs is regulated by Mo-MULV 5' long terminal repeat (LTR).

### Generation of pseudotyped recombinant retroviral vectors encoding

***UP50:*** For retroviral vector production, vector pLXSN-UP50-EGFP or pLXSN-UP50 was transfected into 293FLYA packaging cells using Lipofectamine (Gibco BRL, USA). After 48 hours, supernatant from confluent cultures of viral producer cells was collected, filtered (0.45 µm) and added to 293FLY10A or 293 FLYGALV packaging cells. Transduced cells were grown under G418 selection (400 µg/ml) and individual colonies were collected and screened for EGFP expression using an inverted fluorescent microscope, and were screened for UP50 expression by Western immunoblotting analysis of transduced cell-conditioned medium. The viral titer of each colony was determined via transduction of TE671 cells and titers of ~10⁶ ffu/ml were obtained. Supernatant from colonies with the highest-titers was collected freshly for transduction of EC and SMC.

### Results:

***Viral vector production:*** The vectors listed in Table 1 were utilized to transfer UP50 and VEGF₁₆₅ genes to EC and SMC. Figures 3a, 4a and 4b depict the adenoviral VEGF₁₆₅-GFP, UP50 and UP50-GFP expression vectors, that were constructed and Figures 3b, 4c and 4d depict the retroviral VEGF₁₆₅-GFP UP50 and UP50-GFP expression constructs, according to the present invention.

**Table 1**

| Viral system | Packaging cell line | Gene(s) encoded | Designation | Titer |
|---|---|---|---|---|
| Adenovirus | | GFP | Ad.GFP | 10¹⁰ pfu/ml |
| Adenovirus | | VEGF-IRES-GFP | Ad.VEGF-GFP | 10¹⁰ pfu/ml |
| Adenovirus | | UP50-GFP | Ad.UP50-GFP | 5 x 10¹⁰ pfu/ml |
| Adenovirus | | UP50 | Ad.UP50 | pending |
| Pseudotyped Retrovirus | TEFLYGA | GFP | RetroGFP | 10⁶ ffu |
| Pseudotyped Retrovirus | TEFLYGA | VEGF-IRES-GFP | Retro VEGF-GFP | 5x10⁵ ffu |
| Pseudotyped Retrovirus | 293FLYGA | UP50-IRES-GPF | RetroUP50-GFP | 10⁶ ffu |
| Pseudotyped Retrovirus | 293FLY10A | UP50-IRES-GPF | RetroUP50-GFP | 10⁶ ffu |
| Pseudotyped Retrovirus | 293FLYGA | UP50 | RetroUP50 | 10⁶ ffu |

### EXAMPLE 3

### Expression of pro-endothelializing factors in vascular cells used to seed artificial vascular grafts

There is an ever-increasing need for synthetic grafts having bio-compatible surfaces and high chronic patency rates. The use of synthetic PTFE grafts has gained popularity due to the biocompatibility of the material itself and to its durability. The durability and functionality of synthetic grafts may be enhanced by the coating of luminal surfaces with EC. Such cells constitute the optimal bio-compatible surface, provide long-term protection from thrombosis due to their thrombolytic capacity (11, 12) and prevent neointimal proliferation and inflammatory reaction in synthetic grafts (13).

A powerful approach for optimizing endothelialization of seeded artificial grafts is to employ genetically modified vascular cells overexpressing pro-endothelialization factors. Hence, vascular cells were genetically modified to overexpress such factors, as described below, in order to accelerate seeding of vascular grafts and to maximize post-implantation proliferation of seeded cells, thereby leading to maximal lining of synthetic graft lumenal surfaces with vascular cells.

### Tissue culture of primary human vascular cells:

### Materials and Methods:

Human saphenous vein EC (HSVEC) were harvested from 5 cm-long venous segments of human saphenous vein by collagenase digestion, as previously described (29). Isolated HSVEC were cultured on gelatin-coated dishes containing M199 medium (Gibco BRL, USA) supplemented with 20% pooled human serum, 2 mM L-glutamine (Biological Industries, Israel), 100 units/ml penicillin (Biological Industries, Israel), and 0.1 mg/ml streptomycin (Biological Industries, Israel), 100 µg/ml heparin (Sigma, USA) and 2 ng/ml bFGF (kindly obtained from Prof. Neufeld). Cells from passages 3-9 were collected so as to ensure phenotypic stability, which was monitored by immunohistochemical detection of von Willebrand factor (Zymed, USA). Human saphenous vein SMC (HSVSMC) were cultured by explant outgrowth from human saphenous veins and left internal mammary arteries (HLSMC). Cells were cultured in Dulbecco's Modified Eagles Medium (DMEM) (Gibco BRL, USA) supplemented with 10% pooled human serum, 2 mM L-glutamine, 100 units/ml penicillin, 0.1 mg/ml streptomycin and 2 ng/ml bFGF. SMC were identified by immunohistochemistry analysis using specific anti-smooth muscle α-actin antibodies (Zymed, USA).

### Tissue culture of cell lines:

The packaging cell lines 293-FLYA, 293-FLY10A, 293-FLYGALV and TEFLYGA (obtained from Dr. F.L. Cosset-Lion, France) were grown in DMEM supplemented with 10% FCS (Biological Industries, Israel), 2 mM L-glutamine, 100 units/ml penicillin, 0.1 mg/ml streptomycin, 6 µg/ml blasticidin (Sigma, USA) and 6 µg/ml phleomicin (Sigma, USA).

The packaging cell lines PA317, 293E3 (obtained from Dr. J. Exelrod, Haddasa, Jerusalem) were grown in DMEM supplemented with 10% FCS, 2 mM L-glutamine, 100 units/ml penicillin and 0.1 mg/ml streptomycin.

### Infection of EC and vascular SMC with recombinant adenoviral vectors:

### Materials and Methods:

Endothelial cells and SMC were infected with recombinant adenoviral vectors as follows: the cells were seeded at 70% confluence on fibronectin (Sigma, USA) pre-coated plates (4.5 µg/ml) 20 hours prior to infection and grown in complete medium (M20). On the day of infection the culture medium was replaced with fresh serum-free M199 medium and recombinant virus was added at a multiplicity of infection (MOI) of 3000 (i.e., at 3000 viral particles/cell). The cells were incubated for 90 minutes with gentle tilting every 20 minutes after which the virus-containing medium was replaced with complete medium (M20). The infection rate was monitored by visualization of GFP expression using a fluorescent inverted microscope (TE200 Nikon, Japan) equipped with a fluorescent GFP filter (GFP-LP, Nikon).

### Transduction of EC and SMC with recombinant retroviral vectors:

### Materials and Methods:

Transduction of EC and SMC with retroviral vectors was performed as follows: EC or SMC (passage 4-9) were seeded at 10⁵ cells/35-mm well in plates coated with 4.5 µg/ml fibronectin and grown in complete medium for 24 hours. The cells were pre-conditioned one hour prior to transduction by replacement of the culture medium with serum-free M199 medium containing 0.1 mg/ml DEAE-dextran (Sigma, USA). Following pre-conditioning, the cells were washed three times with phosphate-buffered saline (PBS). Transduction was performed by a 4h incubation of the cells with supernatant freshly collected and filtered (0.45 µl) from virus-producing packaging cells. At the end of the incubation the virus-containing medium was replaced with M20 medium.

### EXAMPLE 4

### Seeding of PTFE grafts with EC

### Materials and Methods:

Human EC were seeded at 50-60% confluence 20 hours prior to transduction. The cells were transduced, as previously described, with retroviral vectors encoding GFP or UP50-GFP, and were used for graft seeding following G418 selection (300 µg/ml) to a obtain a homogeneous cell population. The cells were seeded (2 x 10⁵ cells/1cm²) on fibronectin (4.5 µg/ml) pre-coated ePTFE grafts. The seeding procedure was performed in a horizontal rotating device at 45 rotations/hour for 4 hours at 37°C after which the seeded grafts were placed in M199 medium containing 200 units/ml penicillin and 0.2 mg/ml streptomycin. The efficiency of graft seeding was evaluated by fluorescence microscopy detection of GFP production by seeded cells.

### Results:

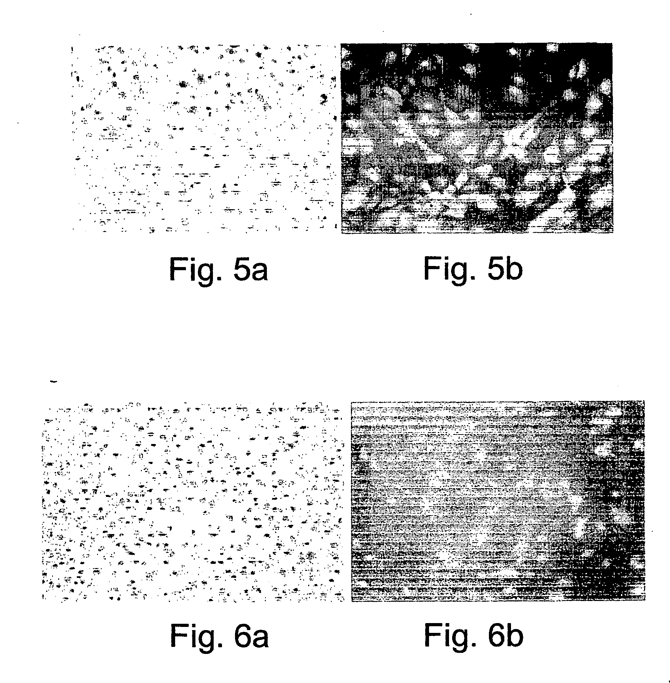

***Expression of Lac Z and VEGF-GFP in human EC transformed with viral vectors and seeded onto synthetic vascular grafts:*** PTFE grafts seeded with human EC infected with Ad.LacZ were found to express β-galactosidase (Figure 5a), as determined via an X-gal colorimetric assay. Grafts seeded with human EC infected with Ad.VEGF-GFP were found to express transgene, as determined by fluorescent microscopy detection of GFP-expressing cells (Figure 5b). Transgene expression was analyzed 24 hours following infection.

PTFE grafts seeded with human EC infected with retroviral vector TGA, encoding nuclear-targeted LacZ (a kind gift from F. Cosset), were found to express β-galactosidase (Figure 6a), as determined via an X-gal colorimetric assay. Grafts seeded with human EC infected with retroVEGF-GFP were found to express transgene as determined by fluorescent microscopy detection of GFP-expressing cells (Figure 6b). Transgene expression was analyzed 24 hours following infection.

These results therefore demonstrated the capacity of the adenoviral and retroviral vectors of the present invention to drive expression of transgenes, such as the pro-endothelialization factor VEGF, in primary human EC seeded onto an artificial graft.

### EXAMPLE 5

### Bone marrow progenitor cell infection with VEGF encoding vectors

### Materials and Methods:

Bone marrow progenitor cells are harvested as previously described (17, 30) and infected with recombinant adenoviral vector encoding VEGF-GFP. Differentiation is monitored in infected cells by morphological analysis and via immunohistochemical detection of von Willebrand factor.

### EXAMPLE 6

### Regulation of gene expression following seeding

### Materials and Methods:

An effector-regulatable expression system is utilized in order to regulate expression of transgene, such as VEGF and UP50, in EC. For example, tetracycline is employed to downregulate VEGF expression and upregulate UP50 expression by expressing these proteins under the regulatory control of promoters which are upregulated and downregulated, respectively, by tetracycline. With such a system, seeded cells express one protein, such as VEGF, in the first week, when cell proliferation is needed, while the use of tetracycline one week following surgery downregulates VEGF expression and upregulates UP50 expression, thereby increasing cell adherence to the grafts. (31).

***Analysis of recombinant protein expression**:* Levels of VEGF protein are assessed by ELISA over a 24-hour period in seeded graft-conditioned supernatant. Similar methods were employed to measure levels of UP50 protein.

### EXAMPLE 7

### Endothelial cell quantitative morphometry

### Materials and Methods:

At the end of the incubation period the grafts are cut longitudinally, examined under fluorescent microscope, photographed and the images processed by an image analysis system (Image pro Plus, Media cybernetics USA).

### EXAMPLE 8

### t-PA and Urokinase measurements

### Materials and Methods:

Level and activity of t-PA are measured in supernatant collected from seeded grafts. Levels of t-PA and urokinase are tested by ELISA while t-PA activity is measured as previously described (32).

### EXAMPLE 9

### Cell recovery from grafts

### Materials and Methods:

Cells are recovered from the PTFE grafts by digestion with EDTA-trypsin. Cells are counted following digestion and re-plated in a tissue culture dish precoated with fibronectin to assess viability. A time from plating to full confluence is recorded. In cells transduced with pseudotyped retroviral vectors, VEGF and UP50 expression is monitored using ELISA and the various methods described in the present invention.

### EXAMPLE 10

### Detection of transgene expression in genetically modified vascular cells

### Materials and Methods:

*RT-PCR analysis of UP50 mRNA transcription:* Total RNA was isolated from EC and SMC 48 hours after transformation with UP50-encoding adenoviral and retroviral vectors, using PURESCRIPT RNA isolation kit (Gentra systems, USA), and RNA concentration was calculated from the absorbance at 260nm.

For cDNA synthesis, 1µg of RNA was mixed with 500ng random hexamers, the mixture was heated at 70°C for 10 minutes and then cooled on ice. The reaction mixture contained 0.4 mM dNTPs, 5 units AMV- reverse transcriptase (RT) (Promega), 5mM DTT, 32 units RNAse-out (Gibco BRL) and RT buffer (Promega) was added to the preheated RNA. The reaction mixture was incubated for 2 hours at 38°C followed by 15 minutes at 95°C. The PCR reaction was performed in a volume of 50µl with 7µl of RT reaction mix, 20 pmole of sense primer: 5'-GAAGATCTTGACATGCC AGGAATAAAAAGGATACTC-3' (SEQ ID NO:2), 20 pmol of anti-sense primer: 5'-GAAGATCTTCAGAATGGGTACTGCGACACATATATCC GCAGTCG-3' (SEQ ID NO:3), 240 mmol dNTPs, 1U Ex-Taq DNA polymerase (Takara, Japan) and reaction buffer provided by the manufacturer. The PCR reaction was performed by incubation at 94°C for 2 minutes followed by 10 cycles of 94°C/30sec -> 50°C/30seconds -> 72°C/60 sec. This was followed by 21 cycles of: 94°C/30sec -> 60°C/30sec -> 72°C/60 sec + 5 sec/cycle -> 72°C/10 min. RT-PCR products were analyzed by electrophoresis in 1% agarose gel.

***Western immunoblot analysis of UP50 and VEGF protein expression:*** Expression of UP50 or VEGF protein by adenovirally or retrovirally transformed EC and SMC was detected by Western immunoblot analysis of transformed cell-conditioned medium. Culture medium was replaced with serum-free medium 24 hours post-transformation and the cells were cultured for an additional 24 hours. Samples of the transformed cell-conditioned medium (CM) (30 µl) were separated by electrophoresis in 10% SDS polyacrylamide gel under reducing conditions. Separated protein was electroblotted onto a nitrocellulose membrane (Schleicher & Schuell). The blots were blocked by incubation blocking solution (TBS containing 0.1% skim milk and 0.3% Tween-20 (TBST)) for 1 hour at room temperature with gentle agitation. Afterwards, the blots were incubated with primary antibody diluted in blocking solution for 2 hours at room temperature. Affinity purified polyclonal rabbit anti-UP50 antibody (#9855, custom made, Sigma, Israel) (1:5000) was used for UP50 detection, and polyclonal rabbit anti-VEGF₁₆₅ antibody (#SC 152 Santa- Cruz, USA) (1:700) was used for VEGF detection.

Following incubation with primary antibody the blots were washed three times with TBST and incubated with anti-rabbit peroxidase-conjugate secondary antibody (Sigma, USA) diluted with TBST for 1 hour at room temperature. After three washes with TBST specific protein was visualized by development of blots with ECL reagents (Sigma, USA) and exposure to X-ray film.

***Immunohistochemical analysis of UP50 expression:*** Adenovirally-infected EC and SMC were seeded on chamber slides (Lab-Tek, USA) pre-coated with fibronectin (4.5 µg/ml), and cultured for 24 hours. Cells were fixed 48h following adenoviral infection by incubation at room temperature for 20 minutes in 4% paraformaldehyde followed by two washes with PBS. The cells were then denatured by heating the slides in a microwave oven for 5 min in 1mM EDTA pH 8.0. The samples were blocked using blocking solution supplied with the Histostain - Plus kit (Zymed, USA) according to manufacturer's instructions. After blocking, the cells were incubated with affinity-purified anti-UP50 (1:50) for 1h at room temperature. The samples were washed 3 times with PBS-T (PBS containing 0.3% Tween-20) and incubated with rhodamine-conjugated goat anti-rabbit IgG antibody (#SC 2091, Santa Cruz, USA) diluted 1:400 in PBS-T for 1 hour. The cells were washed 3 times in PBS-T and covered with mounting medium (H-1000, Vector laboratories, USA). Samples were then visualized by fluorescence scanning confocal (MRC-1024, BioRad) microscopic detection of GFP and rhodamine in the cells.

For immunohistochemical analysis of UP50 in ECM, endothelial cells infected with recombinant adenoviral vector encoding the UP50 gene were induced to generate ECM by addition of dextran to the growth medium. After denudation of the EC layer using 20mM NH₄OH solution, the ECM was subjected to rhodamine-based immunostaining using anti-UP50 antibody.

### Results:

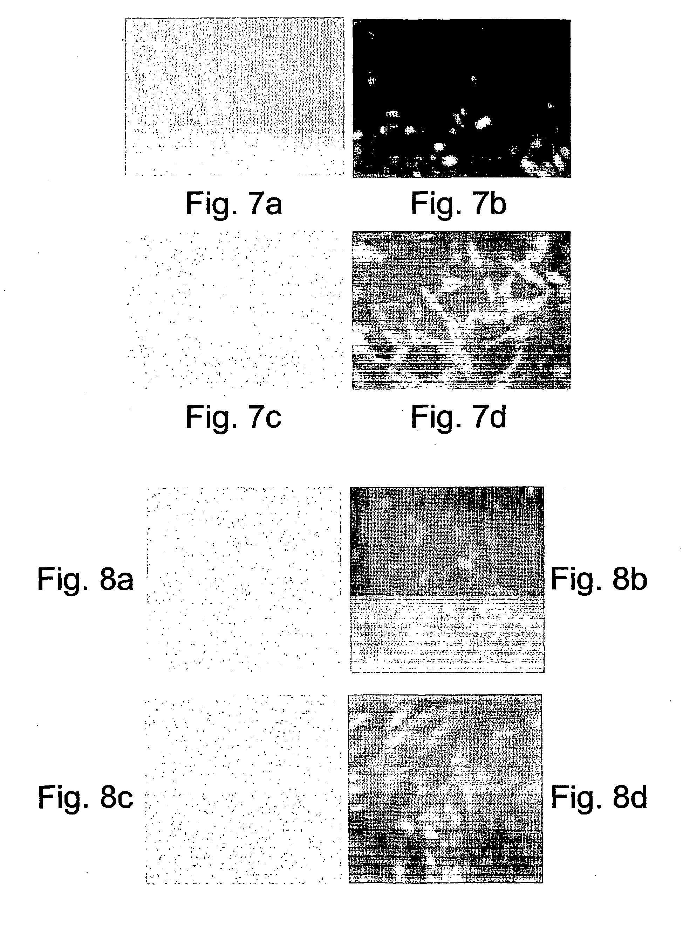

***Analysis of UP50 expression in EC and SMC:*** Infection of EC and SMC by recombinant adenovirus encoding UP50-GFP resulted in transgene expression (Figures 7b and 7d, respectively), as determined by visualization of GFP. Transduction of EC and SMC by retroviral vector encoding UP50-GFP similarly demonstrated production of GFP protein (Figures 8b and 8d).

Transcription of UP50 mRNA was detected by RT-PCR analysis in Ad.UP50-GFP infected EC and SMC (Figures 9a and 9b, respectively) and in EC and SMC genetically modified with retroviral vector encoding UP50-GFP (Figures 10a and 10b, respectively).

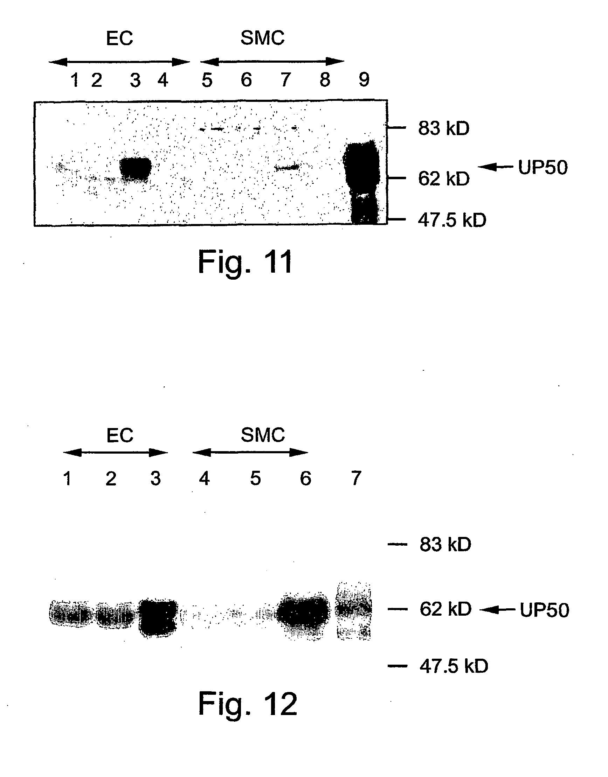

UP50 protein (60 KD) expression was detected by Western immunoblotting analysis following infection of EC and SMC with adenoviral (Figure 11) and retroviral (Figure 12) vectors encoding UP50-GFP.

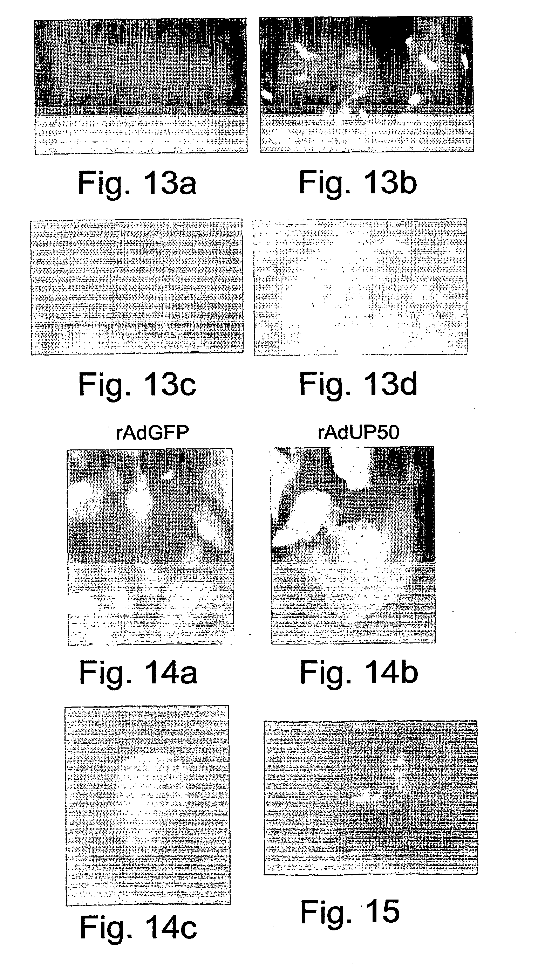

UP50 protein was detected in Ad.UP50-infected EC and SMC, as determined by immunohistochemistry (Figures 13b and 13d, respectively). Cytoplasmic staining was detected in Ad.UP50-GFP infected cells while no staining could be detected in the Ad.GFP infected cells, thus demonstrating high level cytoplasmic expression of UP50.

Confocal microscopy was employed to analyze the sub-cellular location of UP50 within infected EC. Expression of UP50 was detected in Ad.UP50-GFP infected endothelial cells in the cytoplasm and in the cell membrane (Figures 14b and 14c) and was detected as filamentous structures in confluent cells (Figure 14b). The cytoplasmic filamentous expression of UP50 in non-confluent cells implies that UP50 may be associated with the cytoskeleton and may have a function in cell-cell or cell-ECM interactions.

The presence of UP50 protein in ECM produced by EC infected with adenoviral vector encoding UP50-GFP was detected by immunohistochemical analysis (Figure 15).

These results therefore demonstrate that the retroviral and adenoviral expression vectors of the present invention are capable of inducing expression of the cellular adhesion factor UP50 in vascular cells. Therefore, EC and SMC expressing such constructs were employed in functional analyzes aimed at demonstrating the ability of vascular cells expressing UP50 to generate improved synthetic vascular grafts when seeded thereupon.

### EXAMPLE 11

### UP50 and VEGF protein expression by co-cultures of adenovirally-infected EC and SMC

### Materials and Methods:

Co-cultures of adenovirally-infected EC or mixed cultures of adenovirally-infected EC and SMC were cultured for 24 hours following infection in serum-supplemented medium and were further cultured in serum-free medium for an additional 24 hours. Supernatant proteins were separated electrophoretically in 10% SDS polyacrylamide gel and the separated proteins were electroblotted onto nitrocellulose membranes. Blots were incubated with anti-VEGF or anti-UP50 antibodies. Following exposure to peroxidase-conjugated secondary antibody, the blots were developed with ECL reagents and exposed to X-ray film.

### Results:

Co-cultures of Ad.VEGF-GFP infected and either EC or SMC infected with Ad.UP50-GFP demonstrated expression of similar levels of VEGF and UP50 (Figures 16a and 16b, respectively).

These results demonstrated the ability of the method of the present invention to co-produce the EC mitogen VEGF and the EC adhesion factor UP50 and further, to produce these proteins by co-cultures of genetically modified EC and SMC. Hence, the present invention provides an improved method of seeding artificial vascular grafts with endothelial.

### Western immunoblotting analysis of VEGF and UP50 protein expression by co-cultures of retrovirally-infected EC and SMC:

### Materials and Methods:

Co-cultures of EC infected with different retroviruses or mixed cultures of infected EC and SMC were cultured in serum-supplemented medium for 24 hours following infection after which the cells were cultured in serum-free medium for an additional 24 hours. Samples of the growth medium (30 µl) were separated on a 10% SDS polyacrylamide gel and electroblotted onto a nitrocellulose membrane. Blots were incubated with either anti-VEGF or anti-UP50 antibodies. Following exposure to a peroxidase-conjugated secondary antibody the blots were developed with ECL reagents and exposed to X-ray film.

### Results:

Analysis of co-cultures of SMC and EC retrovirally infected to express UP50 and VEGF, respectively, demonstrated co-expression of VEGF and UP50 protein as determined by Western immunoblotting analysis (Figures 17a and 17b).

These results and those described above employing adenoviral vectors therefore demonstrate that the viral expression vectors of the present invention can efficiently drive expression of VEGF and UP50 proteins. These results further demonstrate that both VEGF and UP50 can both be overexpressed in a co-culture of vascular cells genetically modified with either retroviral or adenoviral vectors. Thus, the use of the adenoviral and retroviral expression vectors of the present invention and the use of mixed cultures of genetically modified vascular cells expressing both mitogenic and adhesion-inducing molecules are highly suitable for the production of synthetic vascular grafts possessing long-term durability and patency.

### EXAMPLE 12

### Functional analysis of overexpressed recombinant VEGF and UP50 in vascular cells

### Proliferation assays of EC:

### Materials and Methods:

Endothelial cells (passages 5-11) were seeded at 30% confluence (10⁴ cells/well) in 24-well plates pre-coated with 4.5 µg/ml fibronectin, 24 hours prior to adenoviral infection. The cells were infected with Ad.UP50-GFP, Ad.GFP or Ad.VEGF₁₆₅-GFP. Following 90 minutes of exposure to adenoviral vectors at 37°C, serum-containing medium was added and 16-18 hrs later the medium was substituted with M199 medium containing 2% human serum and 2 ng/ml bFGF. Assays were performed in triplicate and proliferation was measured via XTT colorimetric assay on Day 7 following infection.

### Results:

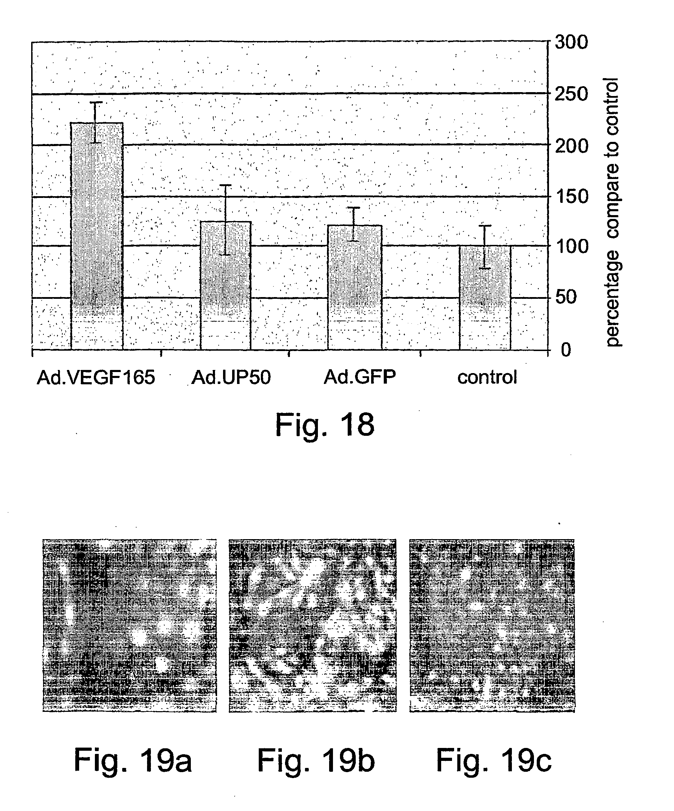

The infection produced almost 100% transgene-expressing cells, as detected by cytoplasmic GFP expression. The results are presented as relative values with respect to non-infected cells. As shown in Figure 18, transgene over-expression of UP50 had no inhibitory or proliferative effect on cell growth rate. Although the values measured in the adenoviral infected groups (Ad.GFP, Ad.UP50-GFP) were slightly higher than the non-infected control group, the effect of UP50 expression was not significant. There was no significant difference between EC infected with Ad.GFP compared to EC infected with Ad.UP50-GFP. Infection with Ad.VEGF₁₆₅-GFP induced significant proliferation compared to other infected EC groups.

### Adhesion of EC to ECM generated by UP50-overexpressing EC:

### Materials and Methods:

***Induction of ECM generation by EC:*** EC were seeded in fibronectin pre-coated 48 well plates (10⁴ cells/well) 24 hours before infection. The cells were infected with Ad.UP50-GFP or Ad.GFP, as previously described. After infection the cells were grown in M20 medium supplemented with 4% dextran 42000 (Sigma USA) for 7 days. Following this infection period, the culture medium was aspirated from the infected cells and the matrix producing cells were denuded by addition of 20 mM NH₄OH for approximately 5 minutes. After cell lysis ECM-coated wells were washed three times with PBS and stored in PBS at 4°C.

Extracellular matrix adhesion assays were performed as follows: EC were detached by incubation in 10mM EDTA solution, the matrix was washed with M199 medium and incubated in CM from Ad.UP50-GFP or Ad.GFP infected or from non-infected EC, respectively for 15 minutes. After this incubation, EC were seeded in the ECM coated wells (2x10⁴ cell per well). The cells seeded on ECM generated by Ad.UP50-GFP infected EC, were incubated in CM from Ad.UP50-GFP infected cells. Cells seeded on ECM generated by Ad.GFP infected cells were incubated with CM collected from Ad.GFP infected cells and the control group of cells seeded on ECM generated by non-infected cells were incubated with non-infected cell-conditioned medium. The cells were incubated for an additional 30 minutes to allow cell adhesion and were then washed with PBS. Quantitation of the remaining adherent cells was performed via XTT colorimetric assay.

Similar experiments were performed utilizing ECM generated from retroUP50-GFP transduced EC.

***Effects of UP50 on cellular physiology and co-cultures:*** It was hypothesized that co-expression of UP50 and VEGF was feasible. Therefore, co-cultures of EC infected to express either VEGF or UP50 were tested for the expression levels of both proteins following mixing Ad.VEGF-GFP infected cells with Ad.UP50-GFP infected EC, as described below.

Endothelial cells transduced with retroviral vector encoding UP50-GFP, GFP or control non-transduced cells were incubated 48 hrs before adenoviral infection. Infection was performed with Ad.VEGF-GFP or Ad.GFP. The cells were incubated for 24 hrs in virus-containing medium after which the medium was replaced with serum-free medium and the cells were incubated for an additional 24 hrs. Samples of the growth medium (30µl) were separated in 10% SDS polyacrylamide gel, separated proteins were electroblotted onto a nitrocellulose membrane and the blots were incubated with either anti-VEGF or anti-UP50 antibodies. Following exposure to a peroxidase-conjugated secondary antibody the blots were developed with ECL reagents and exposed to X-ray film.

### Results:

***Analysis of UP50 and VEGF expression by EC genetically modified sequentially with retroviral and adenoviral vectors:*** Endothelial cells retrovirally transduced to express UP50-GFP and subsequently infected with adenoviral vector encoding VEGF-GFP displayed higher levels of GFP expression than cells infected with retroviral vector encoding UP50-GFP only, as determined by fluorescence microscopy (Figures 19b and 19a, respectively).

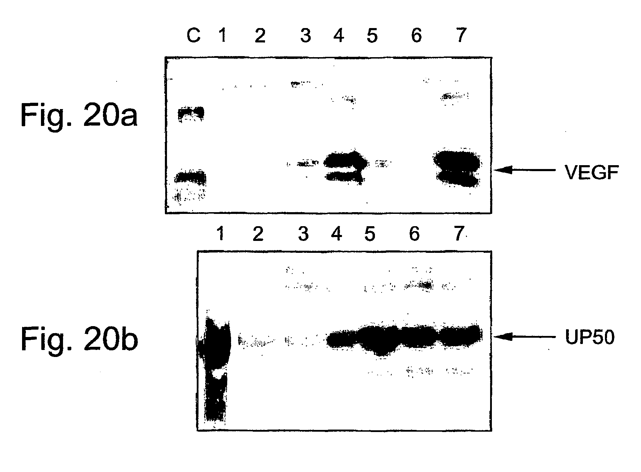

Western immunoblotting analysis of EC retrovirally transduced to express UP50 and subsequently infected with adenoviral vector encoding VEGF genes indicated that such cells could co-express VEGF and UP50 protein (Figures 20a and 20b, respectively).

These results therefore demonstrated that the sequential retroviral transduction and adenoviral infection of EC to co-express both the EC mitogenic and adhesion-inducing proteins VEGF and UP50, respectively, is highly suitable for the production of synthetic vascular grafts possessing long-term durability and patency. The applicability of such a dual viral genetic modification of EC is also supported by the increased levels of GFP produced thereby, as discussed above.

***Effect of UP50 on EC growth in three-dimensional collagen culture - in vitro angiogenesis:*** Seeding of synthetic vascular grafts with EC overexpressing the mitogenic and pro-adhesive factors VEGF and UP50, respectively, is highly desirable for the generation of synthetic vascular grafts with long-term durability and patency. Hence, the combined effects of these factors on the induction of proliferation of EC was examined as follows.

### Materials and Methods:

***In vitro*** angiogenesis in collagen gels was quantitated using adenovirus infected EC spheroids. The generation of EC spheroids was performed as previously described (33). Endothelial cells were suspended in culture medium containing 0.25% (w/v) carboxymethylcellulose and cultured in non-tissue culture-treated round-bottomed 96-well plates (Nunc, Denmark) for 24 h at 37°C, 5% CO₂, during which time the suspended cells formed a single spheroid per well of defined size and cell number (~750 cells/spheroid). The spheroids thus generated were then embedded in collagen gels. Collagen stock solution was prepared prior to use by mixing 8 volumes acidic rat tail collagen extract (equilibrated to 2 mg/ml, 4°C), 1 volume 10x M199 (Gibco BRL, USA). The pH was adjusted to 7.4 by addition of 0.34N NaOH. To prevent sedimentation of spheroids before polymerization of the collagen gel, 1 volume of collagen stock solution was mixed with 1 volume of room-temperature M199 medium containing 40% human serum and 0.5% (w/v) carboxymethylcellulose. Spheroid-containing gels (20-30 spheroids/gel) were rapidly transferred into pre-warmed 24-well plates and allowed to polymerize. The gels were incubated at 37°C with 5% CO₂ and proliferation of EC in the gels was documented by photomicrography using a digital video camera (DXM1200 Nikon, Japan). The sprouting of at least 40 spheroids from each group was analyzed.

### Results:

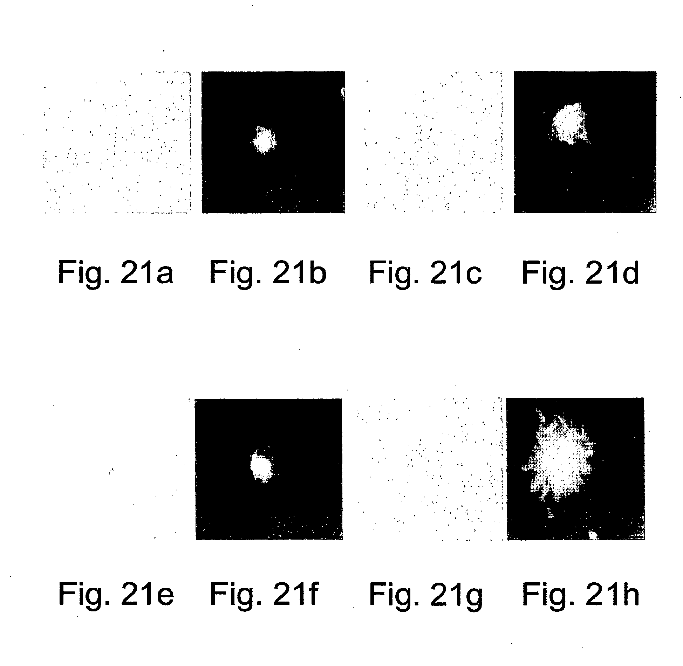

The spheroids of Ad.GFP or AdUP50-GFP infected EC (Figures 21b and 21f, respectively) were found to have a low baseline sprouting activity which was strongly stimulated by addition of exogenous VEGF (Figures 21d and 21h, respectively). The spheroids of Ad.UP50-GFP infected EC (Figure 21f) were found to have a higher baseline sprouting activity than those of Ad.GFP infected EC (Figure 21b).

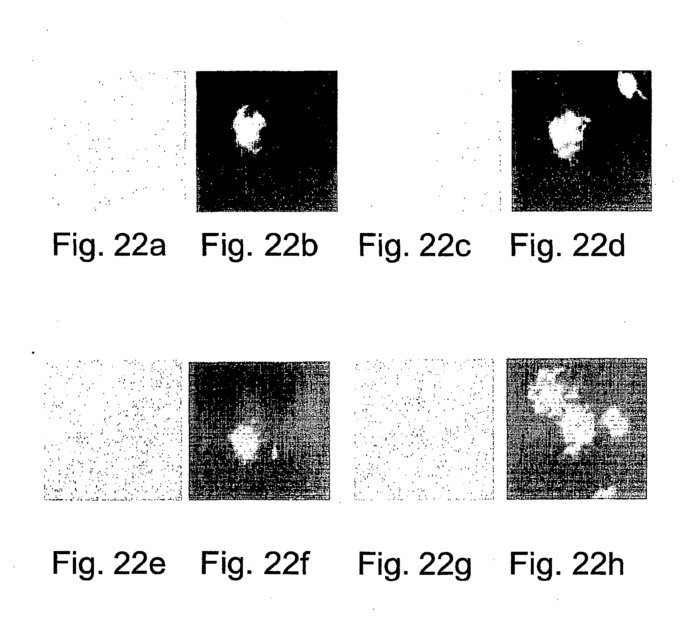

The spheroids of Ad.GFP and Ad.GFP + Ad.UP50-GFP infected EC (Figures 22b and 22f, respectively) were found to have a low baseline sprouting activity which was stimulated in 50% co-cultures with Ad.GFP and Ad.VEGF-infected EC (Figures 22d and 22h, respectively). The highest sprouting levels were observed in the co-culture of Ad.UP50-GFP and Ad.VEGF-GFP infected EC (Figure 22h).

It can be concluded, therefore, that co-overexpression of UP50 with VEGF leads to a synergistic increase in the sprouting of EC as compared to that mediated by VEGF alone.

These results therefore demonstrate that the concomitant co-overexpression of UP50 with VEGF in EC is highly suitable for the production of synthetic vascular grafts possessing long-term durability and patency since EC expressing such a combination of factors display a highly enhanced proliferative capacity.

### EXAMPLE 13

### Effect of UP50 overexpression on adhesion of EC to synthetic grafts in vitro

Synthetic vascular grafts seeded with EC possessing enhanced adhesion characteristics are highly desirable since a major problem of prior art EC-seeded grafts is that these do not efficiently retain the seeded cells. Hence, the effectiveness of overexpressing the EC pro-adhesive factor UP50 to enhance adhesiveness of EC was analyzed as described below.

### Effect of UP50 overexpression on adhesion of in vitro-cultured EC following trypsinization:

### Materials and Methods:

The biological activity of UP50 in Ad.UP50-GFP infected EC was examined by cell retention rate after trypsinization. Endothelial cells were seeded at 70-80% confluence (6-7.5x10⁴ cells/well) in 12 well plates, 24 hours prior to adenoviral infection. The cells were infected (3x10³ pfu per cell), as previously described, with Ad.UP50-GFP or Ad.GFP and non-infected cells served as control. After infection the cells were grown in M20 medium for 4 days. The cells were washed with PBS and adhesion assay was performed by trypsinization of the cells using 0.0025% trypsin containing 0.001% EDTA. After 3 minutes incubation at room temperature, trypsin was neutralized by addition of complete medium (M20). The cells were washed three times with PBS and then M20 (300µl) medium was added to the cells. Quantitation of the remaining adherent cells, as percent of control non trypsinized cells, was determined via colorimetric XTT assay.

### Results:

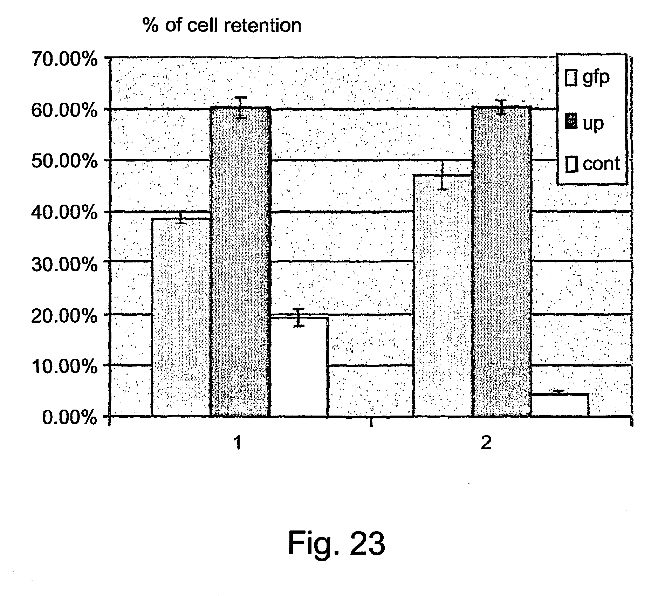

Expression of UP50 following recombinant adenoviral infection significantly increased cell retention (60%) in comparison to Ad.GFP infected cells (40%) or control, non-infected cells (20%) (Figure 23).

### Effect of UP50 overexpression on cell adhesion to ECM:

### Materials and Methods:

EC were detached by 10mM EDTA solution, washed with M199 medium containing 0.1 % BSA (Sigma, USA), 10mM HEPES, and incubated with the CM from Ad.UP50-GFP or Ad.GFP infected or from non-infected EC, respectively for 15 minutes. After incubation the cells were seeded in the ECM coated wells (2x10⁴ cell per well). The cells seeded on ECM generated by Ad.UP50-GFP infected EC, were incubated in CM from Ad.UP50-GFP infected cells. Cells seeded on ECM generated by Ad.GFP infected cells were incubated with CM collected from Ad.GFP infected cells and the control group of cells seeded on ECM generated by non-infected cells were incubated with non-infected cell-conditioned medium. The cells were incubated for additional 30 minutes to allow cell adhesion, than washed with PBS. Quantitation of the remaining adherent cells was performed via colorimetric XTT assay.

### Results:

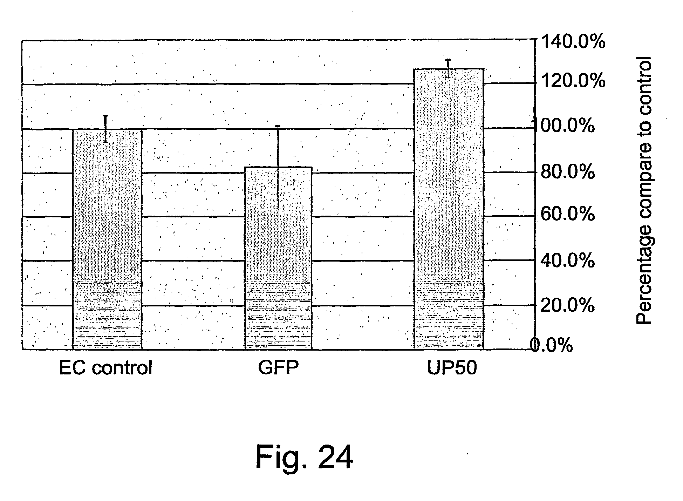

Exposure to secreted UP50 and to ECM-bound UP50, previously shown (Figure 15) resulted in >30% increased adhesion of EC in comparison to the control group (Figure 24).

***Effect of UP50 overexpression on retention of EC under continuous shear stress:*** The adhesion-promoting activity of UP50 on EC was examined by cell retention under continuous shear stress induced shaking culture dishes on a rocking table, as described below.

### Materials and Methods:

Human saphenous vein EC (passage 8-11) were seeded (10⁵ cells per 35mm well) and grown in M20 up to 60-80% confluence. The cells were infected with Ad.UP50-GFP or Ad.GFP (MOI 3000) as described before and non-infected cells served as control. The cells were grown for additional 30 hours to ensure transgene expression before exposing to shear stress. Prior to the experiment 2 wells from each group (Ad.UP50-GFP, Ad.GFP, Control) were harvested using trypsin-EDTA and cells were counted. To cover the cells completely during the rocking 5ml of M20 medium were added to each well. The plates were placed on a rocking table inside the CO₂ incubator and incubated for 20-24 hours under intense rocking (approximately 140 cycles/minute). Following rocking, the cells were washed 5 times with PBS. Cells were harvested using trypsin-EDTA and were counted by hemacytometer. The assay was performed in parallel with cells transduced with retroviral vectors respectively.

### Results:

The results presented in Figure 25a-g (morphometry) and Figure 25h (cell counting) demonstrate that UP50 overexpression by retroviral-transduction induces increased adherence of EC exposed to continuous shear stress. In the UP50-expressing cells 98% of the cells were found to remain adherent to the plate in comparison to GFP-expressing (72%±1%) and non-transduced EC (69±2%).- Similar results were obtained with EC adenovirally-infected to express UP50 (data not shown).

### EXAMPLE 14

### Adhesion of synthetic vascular graft-seeded genetically modified vascular cells exposed to flow conditions

Synthetic vascular grafts can be seeded with vascular cells genetically modified to express EC mitogenic factors to generate grafts possessing long-term durablility and patency. According to the present invention, seeding of vascular grafts is performed with EC adhesion promoting factors to generate vascular grafts whose long-term durability and patency is further enhanced. Thus, the seeding of grafts with such cells and the testing thereof under pulsatile flow conditions was performed as described below.

### Retention of human EC on ePTFE grafts under pulsatile flow

### Materials and Methods:

***Seeding of grafts*:** EC retrovirally transduced to express UP50-GFP were seeded (2 x 10⁵ cells/1 cm²) on fibronectin (4.5 mg/ml) pre-coated ePTFE grafts. The seeding procedure was performed at 37°C for 4 hours in a horizontal rotating device rotating at 45 rotations/h. Seeded grafts were then cultured for 20 hours in M199 medium supplemented with antibiotics.

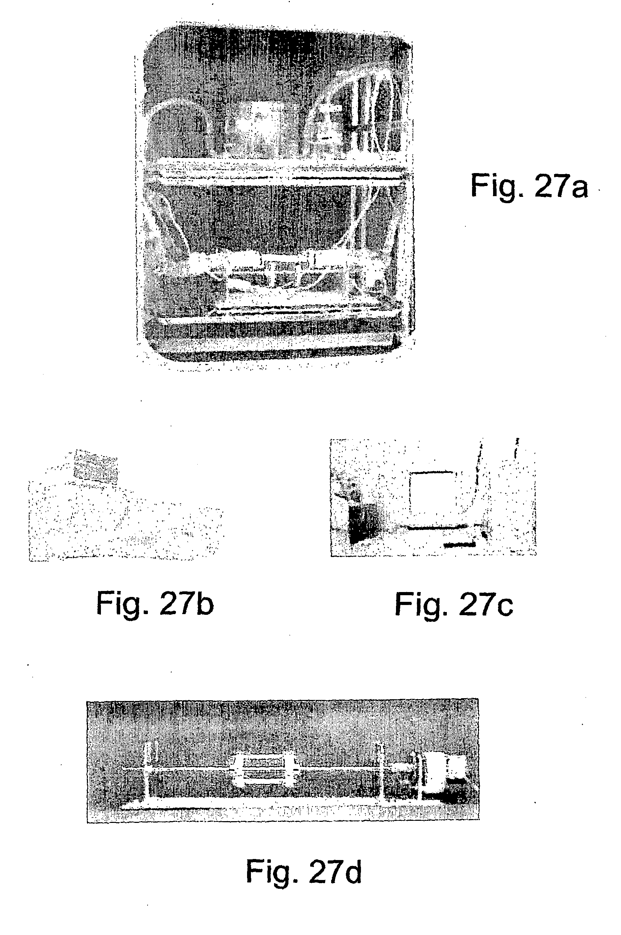

***Description of the flow device*:** The flow system, schematically described in Figure 26, consists of a pulsatile blood pump (Harvard apparatus #1421, USA), flexible tygon tubes, compensation tanks and synthetic grafts, as depicted photographically in Figure 27. System pressure was monitored using a pressure monitor (#742 Mennen Medical, Germany). Flow through the grafts was monitored using a Doppler-based flow system (#T106 Transonic Systems Inc., USA). Both flow and pressure were monitored and recorded online. The pressure and flow data were analyzed by a data acquisition system, which presents online the calculated shear forces inside the grafts. The ePTFE grafts that were used for flow were 5-12 cm long and 6 mm in diameter (GORE Co. USA).

***Flow conditions*:** Seeded grafts were exposed to laminar flow conditions. The flow was adjusted to reach physiological blood pressure of 120/75 (mean of 90 mm Hg) for 45 minutes at flow rate of 150-200 ml/min using 60 pump strokes per minute. The grafts were evaluated using microscopic documentation and morphometric analysis: In the second phase of experiments the grafts was exposed to variable flow conditions for a total of 30 minutes. After exposure to flow conditions, retention of the number of GFP expressing cells was assessed by cutting grafts open longitudinally and fluorescence photomicrographing randomly selected fields of the luminal surfaces.

### Results:

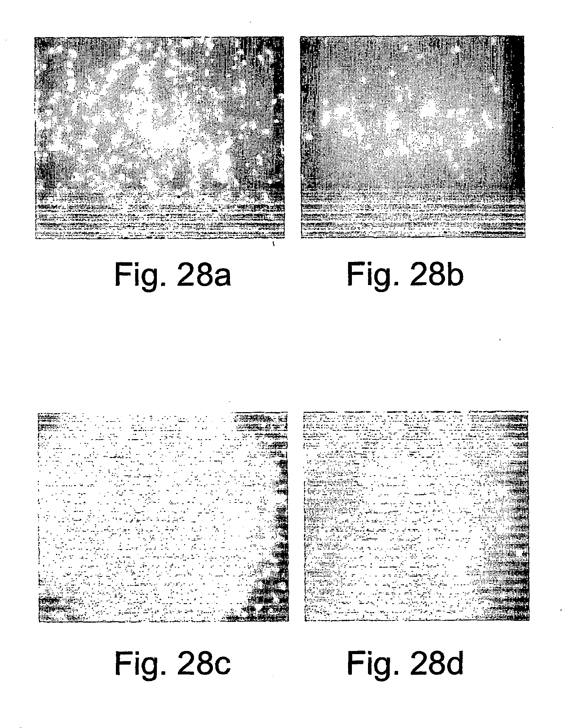

Nearly complete coverage of the grafts was detected by fluorescence microscopy of retroGFP and retroUP50-GFP transduced cells (Figures 28a and 28b, respectively) prior to exposure to pulsatile laminar flow condition, however, following exposure to pulsatile laminar flow conditions, grafts that were coated with EC retrovirally-transduced to express UP50-GFP showed higher cell retention in comparison to grafts coated with EC retrovirally-transduced to express GFP exposed to the same flow conditions (Figures 28b and 28d, respectively).

These findings of increased cell adherence after UP50 gene transfer provide support for the method, described in the present invention, of using seeded grafts with cells overexpressing UP50 for enhancing cell coverage under flow conditions.

### EXAMPLE 15

### Retention of genetically modified EC seeded onto artificial vascular grafts under in vivo flow conditions

***Short-term implantation of ePTFE grafts coated with genetically modified human EC in sheep arteries*:** As a realistic model to test adhesion of human EC genetically modified to express pro-endothelialization factors seeded onto synthetic vascular grafts, grafts seeded with human EC overexpressing such factors were tested *in vivo* in sheep arteries, as follows.

### Materials and Methods:

Small caliber ePTFE grafts (6mm) were seeded with human EC that were genetically modified by retroviral transduction to overexpress GFP, UP50-GFP or VEGF₁₆₅-GFP 36h prior to implantation.

Fasting (12 hours) adult sheep were premedicated with 10 mg diazepam injected intramuscularly and 500-600 mg of intravenously administered sodium pentobarbital and were intubated. Anesthesia was maintained with inhaled 1%-2% isoflurane. Aspirin (600 mg) was administered preoperatively. The monitoring system during the experiment included blood pressure measurement, pulse oxymetry, and ECG. Heparin (300 U/kg) was injected intravenously for systemic anticoagulation following exposure and preparation of arteries for graft implantation. Blood samples were taken during the procedure every 30 minutes to assess the efficacy of heparinization by measuring partial thromboplastin time (PTT).

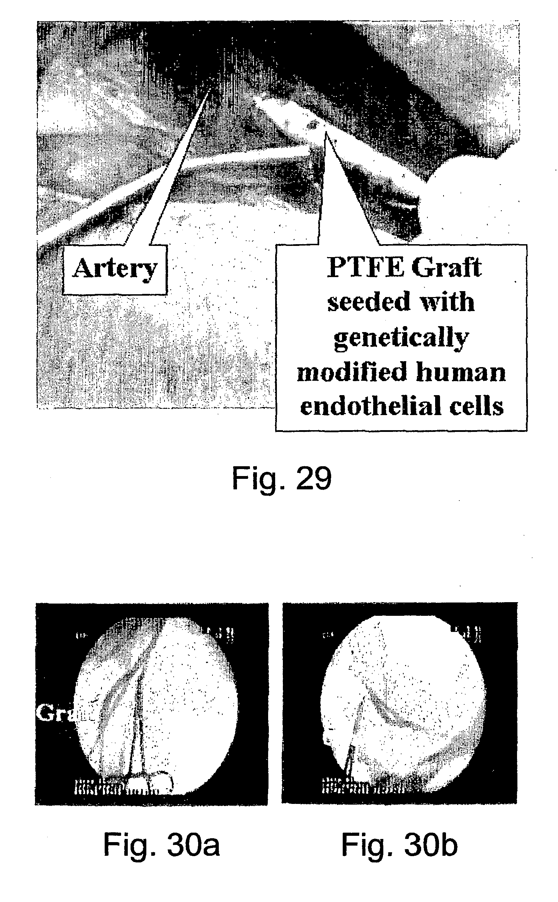

Seeded grafts were implanted end-to-side proximally and distally in the femoral arteries of the sheep by an expert cardiac surgeon as depicted in Figure 29. The grafts were exposed for two hours to blood flow prior to harvesting.

The seeded grafts were then implanted bilaterally end to side in sheep femoral and femoral arteries. On one side of the femoral artery the implanted graft was seeded with retroGFP transduced EC and on the other side the implanted graft was seeded with retroUP50-GFP transduced EC (femoral arteries). In the femoral artery on one side the implanted graft was seeded with retro VEG₁₆₅-GFP transduced EC and on the other side the implanted graft was seeded with retroUP50-GFP transduced EC. Patency of the implanted grafts was assessed 30 minutes following exposure of the implanted grafts to blood flow and prior to graft harvesting by direct palpation, flow measurements using a Doppler flow meter (Transonic Animal Research Flowmeter, NY, USA) and by performing selective angiography (Figure 30).

Flow rates through the femoral grafts were similar in both sides (∼50 ml/min, 38% of femoral blood flow). The flow through the carotid grafts, which was higher at the beginning of the experiment, was bilaterally diminished at the end of two hours due to local thrombosis at the anastomosis site (secondary to surgical complications). The femoral and carotid grafts were harvested two hours following implantation. Both grafts were then harvested and cellular retention on the luminal surfaces of the grafts was analyzed by fluorescence microscopy.

Following graft removal, sheep were sacrificed by intravenous potassium chloride administration.

All experiments were performed according to animal care and experimentation laws of the Technion ITT, Haifa, Israel.

### Results:

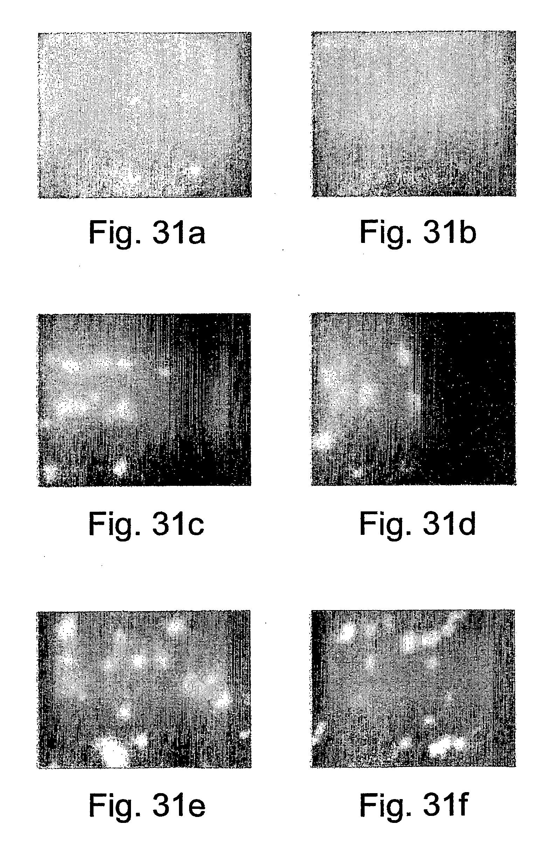

Grafts seeded with cells overexpressing either UP50-GFP or VEGF-GFP clearly displayed a higher number of cells adhering to the grafts following exposure to in vivo blood flow as compared to cells overexpressing GFP only, as determined by fluorescence microscopy visualization of GFP expression (Figure 31). Grafts seeded with cells overexpressing UP50 displayed higher number of adherent cells than the grafts seeded by cells overexpressing GFP or VEGF.

These results, in conjunction with those obtained from *in vitro* adhesion assays performed under either static or flow conditions, as described above, conclusively prove that the genetic modification of EC to overexpress the EC adhesion-promoting factor UP50 leads to a greatly enhanced adhesion of these cells. Therefore, the use of such genetically modified cells to seed synthetic vascular grafts is highly preferable for generating grafts having long-term durability and patency.

### Long-term implantation of artificial vascular grafts seeded with genetically modified autologous EC in sheep:

### Materials and Methods:

Endothelial cells harvested from short venous segments of donor sheep hind limb are expanded in the laboratory and genetically modified with viral vectors. Genetically modified cells are seeded onto grafts and these are implanted end-to-end proximally and distally into the femoral arteries of autologous donor sheep. A graft seeded with genetically modified cells is implanted in one side of a femoral artery, while a graft seeded with cells expressing only a marker gene is implanted in the other side of the femoral artery. Short-term and long-term experiments (3, 7, 30 days) are conducted to allow detection of cell adherence and local thrombosis and inflammation. Long-term graft durability and patency will be assessed on days 7, 30, and 180 (n = 5 for VEGF and n = 5 for UP50) by external measurements of flow using a Doppler flow meter (Transonic Animal Research Flowmeter, NY, USA). At the end of the follow up period, grafts are retrieved and neointimal formation is assessed by cross-sectional morphometric measurement and seeded cell adherence is tested by detection of marker gene expression.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents, patent applications and sequences disclosed therein and/or identified by a Genbank accession number mentioned in this specification are herein incorporated in their entirety by reference into the specification, to the same extent as if each individual publication, patent, patent application or sequence was specifically and individually indicated to be incorporated herein by reference. In addition, citation or identification of any reference in this application shall not be construed as an admission that such reference is available as prior art to the present invention.

### REFERENCES CITED

### (Additional references are cited in the text)

1. VA Study Group 141. Comparative evaluation of prosthetic, reversed, and in situ vein bypass grafts in distal popliteal and tibial-peroneal revascularization. Arch Surg 1988;123:434-8.
2. Londrey GL, Ramsey DE, Hodgson KJ, Barkmeier LD, Sumer DS. Infrapopliteal bypass for severe ischemia: comparison of autogenous vein, composite, and prosthetic grafts. J Vasc J 1991;13 :631-6.
3. Darling RC, Linton RR. Durability of femoropopliteal reconstructions. Am J Surgery 1972;123:472-79.
4. Rutherford RB, Nishikimi N. Graft thrombosis and thromboembolic complications. In Rutherford RB ed. Vascular Surgery. Philadelphia, Pa: WB Saunders Co;1989:501-510.
5. Eickhoff JH, Broome A, Ericsson BF, et al. Four years results of a prospective, randomized clinical trial comparing PTFE and modified human umbilical for below knee femoropopliteal bypass. J Vasc Surg 1987;6:506-511.
6. Meinhart J, Deutsch M, Zilla P. Eight years of clinical endothelial cell transplantation. Closing the gap between prosthetic grafts and vein grafts. ASAIO 1999;43:M515-21.
7. Falk J, Townsend LE, Vogel LM, Boyer M, et al. Improved adherence of genetically modified endothelial cells to small diameter expanded PTFE grafts in canine model. J Vasc Surg 1998;27:902-8.
8. Magometschnigg H, Kadletz M, Vodrazka M, et al. Prospective clinical study with in vitro endothelial cell lining of expanded polytetraflouruethylene grafts in crural repeat reconstruction. J Vasc Surg 1992;15:527-35.
9. Noishiki Y, Tomizawa Y, Yamane Y, Matsumoto A. Autocrine angiogenic vascular prosthesis with bone marrow transplantation. Nature Medicine 1996;2:90-93.
10. Clowes AW. Improving the interface between biomaterials and the blood - the gene therapy approach. Circulation 1996;93:1319-1320.
11. Dichek DA, Anderson J, Kelly AB, Hanson SR, Harker LA. Enhanced in vivo antithrombotic effects of endothelial cells expressing recombinant plasminogen activators transduced with retroviral vectors. Circulation 1996;93:301-309.
12. Gillis-Haegerstrand C, Frebelius S, Haegerstrand A, Swedenberg J. Cultured human endothelial cells seeded on expanded PTFE support thrombin mediated activation of protein C. J Vasc Surg 1996;24:226-34.
13. Pasic M, Muller-Glauser W, Odermatt B, Lachat M, Seifert B, Turina M. Seeding with omental cells prevents late neointimal hyperplasia in small-diameter grafts. Circulation 1995;92:2605-2616.
14. Edelman ER. Vascular tissue engineering. Circ Res 1999;85:11157.
15. Leung DW, Cachians G, Kuang WJ, Goeddel DV, Ferrara N: Vascular endothelial growth factor is a secreted angiogenic factor. Science 1989;246:1306-1309.
16. Hanahan D. Signaling vascular morphogenesis and maintenance. Science 1997;277:48-50.
17. Huang XL. et al., Biochem Biophys Res Commun. 1999, 264:133
18. Sapienza P, di Marzo L, Cucina A, et al. Release of PDGF-BB and bFGF by human endothelial cells seeded on ePTFE vascular grafts. J Surg Res 1998;75:24-9.
19. Van Belle E, Tio FO, Couffinhal T, Maillard L, Passeri J, Isner JM. Stent endothelialization. Circulation 1997;95:438-448.
20. Folkman J: Therapeutic angiogenesis in ischemic limbs [editorial; comment]. Circulation 1998;97:1108-1110
21. Anderson WF. Human gene therapy. Nature 392;25-30.
22. Mulligan RC: The basic science of gene therapy. Science 260:926-932, 1993.
23. Cosset F-L, Russell SJ. Targeting retrovirus entry. Gene therapy 1996;3:946-956.
24. Nakamura T, Ruiz-Lozano P, Lindner V, et al. DANCE, a novel secreted RGD protein expressed in developing, atherosclerotic, and balloon injured arteries. JBC 1999; 274,:22476-22483.
25. Huber TS, Welling TH, Sarkar R, Messina LM, Stanley JC. Effects of retroviral-mediated t-PA gene transfer and expression on adherence and proliferation of canine endothelial cells seeded onto ePTFE. J Vasc Surg 1995;22:795-803.
26. Dunn PF, Newman KD, Jones M, Yamada I, Shayani V, Virmani R, Dichek DA. Seeding of vascular grafts with genetically modified endothelial cells; secretion of recombinant T-PA results in decreased seeded cell retention in vitro and in vivo. Circulation 1996;93:1439-1446.
27. Zilla P, Greisler HP (Eds). Tissue engineering of vascular prosthetic grafts. RG Landes Company, Austin, Texas, USA 1999.
28. He TC. et al., Proc Natl Acad Sci USA 1998, 95:2509
29. Flugelman MY. et al., Circulation Research 1992, 70:348
30. Asahara T. et al., EMBO J. 1999, 18:3964
31. Agha-Mohammadi S. and Lotze MT. J Clinical Investigations 2000, 105:1177
32. Dichek DA. et al., Blood 1991, 77:533
33. Korff T. et al. J Cell Biol. 1998 143:1341
34. Steele JG, Johnson G, Norris WD, Underwood PA. Adhesion and growth of cultured human endothelial cells on perfluorosulphonate: role of vitronectin and fibronectin in cell attachment. Biomaterials 1991 Aug;12(6):531-9.
35. Y. Marois, M.F. Sigot-Luizard and R. Guidoin, Endothelial cell behavior on vascular prosthetic grafts: effect of polymer chemistry, surface structure, and surface treatment, Asaio J. 45 4 (1999), pp. 272-280.
36. G.W. Bos, N.M. Scharenborg, A.A. Poot, G.H. Engbers, J.G. Terlingen, T. Beugeling, W.G. Van Aken and J. Feijen, Adherence and proliferation of endothelial cells on surface-immobilized albumin-heparin conjugate. Tissue Eng. 43 (1998), pp. 267-279.
37. G.W. Bos, N.M. Scharenborg, A.A. Poot, G.H. Engbers, T. Beugeling, W.G. van Aken and J. Feijen, Proliferation of endothelial cells on surface-immobilized albumin-heparin conjugate loaded with basic fibroblast growth factor. J. Biomed. Mater. Res. 44 3 (1999), pp. 330-340.
38. H.M. Carr, R. Vohra, H. Sharma, J.V. Smyth, O.B. Rooney, P.D. Dodd and M.G. Walker, Endothelial cell seeding kinetics under chronic flow in prosthetic grafts. Ann. Vasc. Surg. 10 5 (1996), pp. 469-475.
39. R. Vohra, G.J. Thomson, H.M. Carr, H. Sharma and M.G. Walker, Comparison of different vascular prostheses and matrices in relation to endothelial seeding. Br. J Surg. 78 4 (1991), pp. 417-420.
40. G.B. Koveker, L.M. Graham, W.E. Burkel, R. Sell, T.W. Wakefield, K. Dietrich and J.C. Stanley, Extracellular matrix preparation of expanded polytetrafluoroethylene grafts seeded with endothelial cells: influence on early platelet deposition, cellular growth, and luminal prostacyclin release. Surgery 109 3 Part 1 (1991), pp. 313-319.
41. J.E. Hasson, D.H. Wiebe, J.B. Sharefkin, P.A. D'Amore and W.M. Abbott, Use of tritiated thymidine as a marker to compare the effects of matrix proteins on adult human vascular endothelial cell attachment: implications for seeding of vascular prostheses. Surgery 100 5 (1986), pp. 884-892.
42. R.B. Patterson, J.D. Keller, E.B. Silberstein and R.F. Kempczinski, A comparison between fibronectin and Matrigel pretreated ePTFE vascular grafts. Ann. Vasc. Surg. 3 2 (1989), pp. 160-166.
43. H.M. Carr, R. Vohra, M. Welch, O.B. Rooney, H. Sharma and M.G. Walker, fibronectin binding to gelatin-impregnated Dacron (Gelseal) prostheses. Artif. Organs 16 4 (1992), pp. 342-345.
44. A. Sank, K. Rostami, F. Weaver, D. Ertl, A. Yellin, M. Nimni and T.L. Tuan, New evidence and new hope concerning endothelial seeding of vascular grafts. Am. J. Surg. 164 3 (1992), pp. 199-204.
45. G.J. Thomson, R.K. Vohra, M.H. Carr and M.G. Walker, Adult human endothelial cell seeding using expanded polytetrafluoroethylene vascular grafts: a comparison of four substrates. Surgery 109 1 (1991), pp. 20-27.
46. Braddock M, Schwachtgen JL, Houston P, Dickson MC, Lee MJ, Campbell CJ. Fluid Shear Stress Modulation of Gene Expression in Endothelial Cells. News Physiol Sci. 1998 Oct; 13:241-246.
47. Morimoto M, Kume N, Miyamoto S, Ueno Y, Kataoka H, Minami M, Hayashida K, Hashimoto N, Kita T. Lysophosphatidylcholine induces early growth response factor-1 expression and activates the core promoter of pdgf-a chain in vascular endothelial cells. Arterioscler Thromb Vasc Biol. 2001 May;21(5):771-6.
48. Ogasawara A, Arakawa T, Kaneda T, Takuma T, Sato T, Kaneko H, Kumegawa M, Hakeda Y. Fluid Shear Stress-induced Cyclooxygenase-2 Expression Is Mediated by C/EBP beta, cAMP-response Element-binding Protein, and AP-1 in Osteoblastic MC3T3-E1 Cells. J Biol Chem. 2001 Mar 9;276(10):7048-54.
49. Lin K, Hsu PP, Chen BP, Yuan S, Usami S, Shyy JY, Li YS, Chien S. Free in PMC. Molecular mechanism of endothelial growth arrest by laminar shear stress. Proc Nat1 Acad Sci USA. 2000 Aug 15;97(17):9385-9.
50. Houston P, White BP, Campbell CJ, Braddock M. Delivery and expression of fluid shear stress-inducible promoters to the vessel wall: applications for cardiovascular gene therapy. Hum Gene Ther. 1999 Dec 10;10(18):3031-44.
51. Nakamura T. et al., J Biol Chem. 1999, 274:22476.
52. Shattil, SJ., and Ginsberg, MH. J. Clin. Invest. 1997, 100:S91.
53. Hynes, RO., Cell 1992, 69:11.
54. Doolittle RF. et al., Nature 1984, 307:558.
55. Selander-Sunnerhagen, M. et al., J. Biol. Chem. 1992, 267:19642.
56. Apella E. et al. FEBS Lett 1988 231:1.
57. Kowal RC. et al., Circ Res. 1999, 84:1166.

## Claims

1. An artificial vascular graft comprising a synthetic tubular element having a luminal surface being coated with a plurality of endothelial cells and/or smooth muscle cells being genetically transformed to express at least one cell proliferating growth factor and at least one cellular adherence factor.

2. The artificial vascular graft of claim 1, wherein a first portion of said plurality of endothelial cells and/or smooth muscle cells is genetically transformed to express said at least one cell proliferating growth factor and further wherein a second portion of said plurality of endothelial cells and/or smooth muscle cells is genetically transformed to express said at least one cellular adherence factor,

3. The artificial vascular graft of claim 1, wherein said luminal surface is of a substance selected from the group consisting of polytetrafluoroethylene (PTFE), expanded polytetrafluoroethylene (ePTFE), polyester fibers and, Dacron^{™}.

4. The artificial vascular graft of claim 4, wherein said inner cross sectional area of said synthetic tubular element is within a range of about 7 to 700 mm².

5. The artificial vascular graft of claim 1, wherein said plurality of endothelial cells are derived from a source selected from the group consisting of a segment of a vein, bone marrow progenitor cells, peripheral blood stem cells and circulating endothelial cells.

6. The artificial vascular graft of claim 1, wherein said plurality of endothelial cells and/or smooth muscle cells are derived from a human or an animal donor.

7. The artificial vascular graft of claim 1, wherein said plurality of endothelial cells form a confluent monolayer at said inner luminal surface.

8. The artificial vascular graft of claim 1, wherein said at least one cell proliferating growth factor is selected from the group consisting of VEGF, acidic or basic FGF and HGF.

9. The artificial vascular graft of claim 1, wherein said at least one cellular adherence factor is UP50, DANCE, vitronectin, albumin, collagen I, collagen IV, fibronectin and Laminin.

10. The artificial vascular graft of claim 1, wherein said plurality of endothelial cells and/or smooth muscle cells are further genetically transformed to express at least one marker polypeptide.

11. The artificial vascular graft of claim 1, wherein said synthetic tubular element having said luminal surface is coated with endothelial cells and smooth muscle cells, at least one of said endothelial cells and smooth muscle cells being genetically transformed to express at least one cell proliferating growth factor and at least one cellular adherence factor.

12. The artificial vascular graft of claim 1, wherein said synthetic tubular element having said luminal surface is coated with a plurality of endothelial cells being genetically transformed to express UP50 and VEGF,

13. The artificial vascular graft of claim 1, wherein said synthetic tubular element having said luminal surface is coated with a plurality of smooth muscle cells being genetically transformed to express UP50 and VEGF.

14. The artificial vascular graft of claim 1, wherein said plurality of endothelial cells and/or smooth muscle cells are genetically transformed to express UP50 and a cell growth proliferating factor selected from the group consisting of acidic or basic FGF and HGF.

15. The artificial vascular graft of claim 1, wherein said luminal surface is coated with said plurality of endothelial cells and an exterior surface of said synthetic tubular element is coated with said plurality of smooth muscle cells.

16. The artificial vascular graft of claim 15, wherein said plurality of endothelial cells are genetically transformed to express at least one cell proliferating growth factor and at least one cellular adherence factor.

17. The artificial vascular graft of claim 15, wherein said plurality of smooth muscle cells are genetically transformed to express at least one cellular adherence factor.

18. A transformed endothelial cell or smooth muscle cell being genetically transformed to express at least one cell proliferating growth factor and at least one cellular adherence factor.

19. The transformed endothelial cell or smooth muscle cell of claim 18, being further genetically transformed to express at least one marker polypeptide.

20. The transformed endothelial cell or smooth muscle cell of claim 18, wherein said endothelial cell is derived from a source selected from the group consisting of a segment of a vein, and bone marrow progenitor cells, peripheral blood stem cells and circulating endothelial cells.

21. The transformed endothelial cell or smooth muscle cell of claim 18, wherein said endothelial cell or smooth muscle cell is derived from a source selected from the group consisting of a human donor and an animal source.

22. The transformed endothelial cell or smooth muscle cell of claim 18, wherein said cellular proliferating growth factor is selected from the group consisting of VEGF, acidic or basic FGF and HGF.

23. The transformed endothelial cell or smooth muscle cell of claim 18, wherein said cellular adherence factor is UP50, DANCE, vitronectin, albumin, collagen I, collagen IV, fibronectin and Laminin.

24. A method of producing an artificial vascular graft, the method comprising:
(a) genetically transforming endothelial cells and/or smooth muscle cells to express at least one cell proliferating growth factor and at least one cellular adherence factor; and
(b) culturing said endothelial cells and/or smooth muscle cells within a synthetic tubular element having a luminal surface until sufficient coating of said luminal surface with said endothelial cells and/or smooth muscle cells is achieved,

25. The method of claim 24, wherein step (a) is effected by genetically transforming a first portion of said endothelial cells and/or smooth muscle cells to express said at least one cell proliferating growth factor and genetically transforming a second portion of said endothelial cells and/or smooth muscle cells to express said at least one cellular adherence factor.

26. The method of claim 24, wherein said step (a) precedes said step (b).

27. The method of claim 24, wherein said step (b) precedes said step (a).

28. The method of claim 24, wherein said endothelial cells are derived from a source selected from the group consisting of a segment of a vein, and bone marrow progenitor cells, peripheral blood stem cells and circulating endothelial cells.

29. The method of claim 24, further comprising subjecting said luminal surface of said artificial graft to flow forces.

30. The method of claim 24, wherein said at least one cell proliferating growth factor is VEGF and said at least one cellular adherence factor is UP50.

31. A nucleic acid expression construct or construct system comprising:
(a) a first polynucleotide segment encoding a cell proliferating growth factor; and
(b) a second polynucleotide segment encoding cellular adherence factor.

32. The nucleic acid expression construct or construct system of claim 31, further comprising at least one promoter sequence being for directing the expression of at least one of said first and said second polynucleotide segments,

33. The nucleic acid construct or construct system of claim 32, wherein said first polynucleotide segment is transcriptionally linked to said second polynucleotide segment whereas said first and said second polynucleotide segment are under the transcriptional control of a single promoter sequence of said at least one promoter sequence.

34. The nucleic acid construct or construct system of claim 33, further comprising a linker sequence being interposed between said first and said second polynucleotide segments.

35. The nucleic acid construct or construct system of claim 34, where said linker sequence is selected from the group consisting of IRES and a protease cleavage recognition site.

36. The nucleic acid expression construct or construct system of claim 31, wherein said at least one promoter is functional in eukaryotic cells.

37. The nucleic acid expression construct or construct system of claim 31, wherein said at least one promoter is selected from the group consisting of a constitutive promoter, an inducible promoter and a tissue specific promoter.

38. The nucleic acid expression construct or construct system of claim 31, further comprising;
(c) a first promoter sequence being for directing the expression of said first polynucleotide segment; and
(d) a second promoter sequence being for directing the expression of said second polynucleotide segment.

39. The nucleic acid expression construct or construct system of claim 38, wherein said first promoter and said second promoter are selected from the group consisting of a constitutive promoter, an inducible promoter and a tissue specific promoter.

40. The nucleic acid expression construct or construct system of claim 39, wherein said inducible promoter are regulatable by same effector molecule.

41. The nucleic acid expression construct or construct system of claim 31, further comprising at least one additional polynucleotide segment encoding a marker polypeptide.

42. The nucleic acid expression construct or construct system of claim 41, wherein said marker polypeptide is selected from the group consisting of a selection polypeptide and a reporter polypeptide.

43. The nucleic acid expression construct or construct system of claim 41, wherein said at least one additional polynucleotide segment is transcriptionally linked to said at least one of said first and said second polynucleotide segments,

44. The nucleic acid constructs or construct system of claim 41, wherein said at least one additional polynucleotide segment is transcriptionally linked to said at least one of said first and said second polynucleotide segments via linker segment.

45. The nucleic acid construct or construct system of claim 44, wherein said linker sequence is selected from the group consisting of IRES and a protease cleavage recognition site.

46. The nucleic acid construct or construct system of claim 41, wherein said at least one additional polynucleotide segment is translationally fused to at least one of said first and said second polynucleotide segments.

47. A kit comprising a stand for engaging at least one tube, said at least one tube including:
(a) a first polynucleotide encoding a cell proliferating growth factor; and
(b) a second polynucleotide encoding cellular adherence factor.

48. The kit of claim 47, wherein said first polynucleotide is in a first nucleic acid expression construct and said second polynucleotide is in a second nucleic acid expression construct.

49. A method of producing genetically transformed endothelial cells and/or smooth muscle cells, the method comprising:
transforming said endothelial cells and/or smooth muscle cells which have been obtained from a mammalian tissue source, to express at least one cell proliferating growth factor and at least one cellular adherence factor,

50. The artificial vascular graft of claim 1, for use in the treatment of vascular disease in a subject in need thereof.

## Patentansprüche

1. Künstliches Gefäßtransplantat, umfassend ein synthetisches Röhrenelement mit einer Lumenoberfläche, die mit einer Mehrzahl von Endothelzellen und/oder Eingeweidemuskelzellen beschichtet ist, die genetisch transformiert sind, um wenigstens einen Zellproliferations-Wachstumsfaktor und wenigstens einen Zelladhärenzfaktor zu exprimieren.

2. Künstliches Gefäßtransplantat nach Anspruch 1, wobei ein erster Teil der Mehrzahl von Endothelzellen und/oder Eingeweidemuskelzellen genetisch transformiert ist, um den wenigstens einen Zellproliferations-Wachstumsfaktor zu exprimieren, und wobei ferner ein zweiter Teil der Mehrzahl von Endothelzellen und/oder Eingeweidemuskelzellen genetisch transformiert ist, um den wenigstens einen Zelladhärenzfaktor zu exprimieren.

3. Künstliches Gefäßtransplantat nach Anspruch 1, wobei die Lumenoberfläche aus einer Substanz ist, die aus der Gruppe bestehend aus Polytetrafluorethylen (PTFE), geschäumtem Polytetrafluorethylen (ePTFE), Polyesterfasern und Dacron^{™} ausgewählt ist.

4. Künstliches Gefäßtransplantat nach Anspruch 4, wobei die innere Querschnittfläche des synthetischen Röhrenelements innerhalb eines Bereichs von etwa 7 bis 700 mm² liegt.

5. Künstliches Gefäßtransplantat nach Anspruch 1, wobei die Mehrzahl von Endothelzellen von einer Quelle stammt, die aus der Gruppe bestehend aus einem Segment einer Vene, Knochenmark-Vorläuferzellen, Stammzellen des peripheren Blutes und zirkulierenden Endothelzellen ausgewählt ist.

6. Künstliches Gefäßtransplantat nach Anspruch 1, wobei die Mehrzahl von Endothelzellen und/oder Eingeweidemuskelzellen von einem menschlichen oder einem tierischen Spender stammt.

7. Künstliches Gefäßtransplantat nach Anspruch 1, wobei die Mehrzahl von Endothelzellen eine zusammenwachsende einlagige Schicht auf der inneren Lumenoberfläche bildet.

8. Künstliches Gefäßtransplantat nach Anspruch 1, wobei der wenigstens eine Zellproliferations-Wachstumsfaktor aus der Gruppe bestehend aus VEGF und saurem oder basischem FGF und HGF ausgewählt ist.

9. Künstliches Gefäßtransplantat nach Anspruch 1, wobei es sich bei dem wenigstens einen Zelladhärenzfaktor um UP50, DANCE, Vitronectin, Albumin, Collagen I, Collagen IV, Fibronectin und Laminin handelt.

10. Künstliches Gefäßtransplantat nach Anspruch 1, wobei die Mehrzahl von Endothelzellen und/oder Eingeweidemuskelzellen ferner genetisch transformiert ist, um wenigstens ein Marker-Polypeptid zu exprimieren.

11. Künstliches Gefäßtransplantat nach Anspruch 1, wobei das synthetische Röhrenelement mit der Lumenoberfläche mit Endothelzellen und Eingeweidemuskelzellen beschichtet ist, wobei wenigstens eines von den Endothelzellen und Eingeweidemuskelzellen genetisch transformiert ist, um wenigstens einen Zellproliferations-Wachstumsfaktor und wenigstens einen Zelladhärenzfaktor zu exprimieren.

12. Künstliches Gefäßtransplantat nach Anspruch 1, wobei das synthetische Röhrenelement mit der Lumenoberfläche mit einer Mehrzahl von Endothelzellen beschichtet ist, die genetisch transformiert sind, um UP50 und VEGF zu exprimieren.

13. Künstliches Gefäßtransplantat nach Anspruch 1, wobei das synthetische Röhrenelement mit der Lumenoberfläche mit einer Mehrzahl von Eingeweidemuskelzellen beschichtet ist, die genetisch transformiert sind, um UP50 und VEGF zu exprimieren.

14. Künstliches Gefäßtransplantat nach Anspruch 1, wobei die Mehrzahl von Endothelzellen und/oder Eingeweidemuskelzellen genetisch transformiert ist, um UP50 und einen Zellwachstums-Proliferationsfaktor zu exprimieren, der aus der Gruppe bestehend aus saurem oder basischem FGF und HGF ausgewählt ist.

15. Künstliches Gefäßtransplantat nach Anspruch 1, wobei die Lumenoberfläche mit der Mehrzahl von Endothelzellen beschichtet ist und eine Außenfläche des synthetischen Röhrenelements mit der Mehrzahl von Eingeweidemuskelzellen beschichtet ist.

16. Künstliches Gefäßtransplantat nach Anspruch 15, wobei die Mehrzahl von Endothelzellen genetisch transformiert ist, um wenigstens einen Zellproliferations-Wachstumsfaktor und wenigstens einen Zelladhärenzfaktor zu exprimieren.

17. Künstliches Gefäßtransplantat nach Anspruch 15, wobei die Mehrzahl von Eingeweidemuskelzellen genetisch transformiert ist, um wenigstens einen Zelladhärenzfaktor zu exprimieren.

18. Transformierte Endothelzelle oder Eingeweidemuskelzelle, die genetisch transformiert ist, um wenigstens einen Zellproliferations-Wachstumsfaktor und wenigstens einen Zelladhärenzfaktor zu exprimieren.

19. Transformierte Endothelzelle oder Eingeweidemuskelzelle nach Anspruch 18, die ferner transformiert ist, um wenigstens ein Marker-Polypeptid zu exprimieren.

20. Transformierte Endothelzelle oder Eingeweidemuskelzelle nach Anspruch 18, wobei die Endothelzelle von einer Quelle stammt, die aus der Gruppe bestehend aus einem Segment einer Vene und Knochenmark-Vorläuferzellen, Stammzellen des peripheren Blutes und zirkulierenden Endothelzellen ausgewählt ist.

21. Transformierte Endothelzelle oder Eingeweidemuskelzelle nach Anspruch 18, wobei die Endothelzelle oder Eingeweidemuskelzelle von einer Quelle stammt, die aus der Gruppe bestehend aus einem menschlichen Spender und einer tierischen Quelle ausgewählt ist.

22. Transformierte Endothelzelle oder Eingeweidemuskelzelle nach Anspruch 18, wobei der Zellproliferations-Wachstumsfaktor aus der Gruppe bestehend aus VEGF und saurem oder basischem FGF und HGF ausgewählt ist.

23. Transformierte Endothelzelle oder Eingeweidemuskelzelle nach Anspruch 18, wobei es sich bei dem Zelladhärenzfaktor um UP50, DANCE, Vitronectin, Albumin, Collagen I, Collagen IV, Fibronectin und Laminin handelt.

24. Verfahren zur Herstellung eines künstlichen Gefäßtransplantats, wobei das Verfahren umfasst:
(a) genetisches Transformieren von Endothelzellen und/oder Eingeweidemuskelzellen, um wenigstens einen Zellproliferations-Wachstumsfaktor und wenigstens einen Zelladhärenzfaktor zu exprimieren; und
(b) Kultivieren der Endothelzellen und/oder Eingeweidemuskelzellen innerhalb eines synthetischen Röhrenelements mit einer Lumenoberfläche, bis eine ausreichende Beschichtung der Lumenoberfläche mit den Endothelzellen und/oder Eingeweidemuskelzellen erreicht wird.

25. Verfahren nach Anspruch 24, wobei Schritt (a) durch genetisches Transformieren eines ersten Teils der Endothelzellen und/oder Eingeweidemuskelzellen, um den wenigstens einen Zellproliferations-Wachstumsfaktor zu exprimieren, und genetisches Transformieren eines zweiten Teils der Endothelzellen und/oder Eingeweidemuskelzellen erfolgt, um den wenigstens einen Zelladhärenzfaktor zu exprimieren.

26. Verfahren nach Anspruch 24, wobei Schritt (a) dem Schritt (b) vorangeht.

27. Verfahren nach Anspruch 24, wobei Schritt (b) dem Schritt (a) vorangeht.

28. Verfahren nach Anspruch 24, wobei die Endothelzellen von einer Quelle stammen, die aus der Gruppe bestehend aus einem Segment einer Vene und Knochenmark-Vorläuferzellen, Stammzellen des peripheren Blutes und zirkulierenden Endothelzellen ausgewählt ist.

29. Verfahren nach Anspruch 24, ferner umfassend ein Aussetzen der Lumenoberfläche des künstlichen Transplantats Strömungskräften.

30. Verfahren nach Anspruch 24, wobei es sich bei dem wenigstens einen Zellproliferations-Wachstumsfaktor um VEGF und bei dem wenigstens einen Zelladhärenzfaktor um UP50 handelt.

31. Nucleinsäure-Expressionskonstrukt oder -konstruktsystem, umfassend:
(a) ein erstes Polynucleotidsegment, das einen Zellproliferations-Wachstumsfaktor codiert; und
(b) ein zweites Polynucleotidsegment, das einen Zelladhärenzfaktor codiert.

32. Nucleinsäure-Expressionskonstrukt oder -konstruktsystem nach Anspruch 31, ferner umfassend wenigstens eine Promotorsequenz, die zum Richten der Expression wenigstens eines der ersten und der zweiten Polynucleotidsegmente dient.

33. Nucleinsäure-Expressionskonstrukt oder -konstruktsystem nach Anspruch 32, wobei das erste Polynucleotidsegment transkriptionell an das zweite Polynucleotidsegment gebunden ist, während das erste und das zweite Polynucleotidsegment unter der Transkriptionskontrolle einer einzelnen Promotorsequenz der wenigstens einen Promotorsequenz stehen.

34. Nucleinsäure-Expressionskonstrukt oder -konstruktsystem nach Anspruch 33, ferner umfassend eine Linkersequenz, die zwischen den ersten und den zweiten Polynucleotidsegmenten eingefügt ist.

35. Nucleinsäure-Expressionskonstrukt oder -konstruktsystem nach Anspruch 34, wobei die Linkersequenz aus der Gruppe bestehend aus IRES und einer Proteasenspaltungs-Erkennungssequenz ausgewählt ist.

36. Nucleinsäure-Expressionskonstrukt oder -konstruktsystem nach Anspruch 31, wobei der wenigstens eine Promotor in eukaryotischen Zellen wirkt.

37. Nucleinsäure-Expressionskonstrukt oder -konstruktsystem nach Anspruch 31, wobei der wenigstens eine Promotor aus der Gruppe bestehend aus einem konstitutiven Promotor, einem induzierbaren Promotor und einem gewebespezifischen Promotor ausgewählt ist.

38. Nucleinsäure-Expressionskonstrukt oder -konstruktsystem nach Anspruch 31, ferner umfassend:
(a) eine erste Promotorsequenz, die zum Richten der Expression des ersten Polynucleotidsegments dient; und
(b) eine zweite Promotorsequenz, die zum Richten der Expression des zweiten Polynucleofidsegments dient.

39. Nucleinsäure-Expressionskonstrukt oder -konstruktsystem nach Anspruch 38, wobei der erste Promotor und der zweite Promotor aus der Gruppe bestehend aus einem konstitutiven Promotor, einem induzierbaren Promotor und einem gewebespezifischen Promotor ausgewählt sind.

40. Nucleinsäure-Expressionskonstrukt oder -konstruktsystem nach Anspruch 39, wobei die induzierbaren Promotoren durch dasselbe Effektormolekül regulierbar sind.

41. Nucleinsäure-Expressionskonstrukt oder -konstruktsystem nach Anspruch 31, ferner umfassend wenigstens ein zusätzliches Polynucleotidsegment, das ein Marker-Polypeptid codiert.

42. Nucleinsäure-Expressionskonstrukt oder -konstruktsystem nach Anspruch 41, wobei das Marker-Polypeptid aus der Gruppe bestehend aus einem Selektions-Polypeptid und einem Reporter-Polypeptid ausgewählt ist.

43. Nucleinsäure-Expressionskonstrukt oder -konstruktsystem nach Anspruch 41, wobei das wenigstens eine zusätzliche Polypeptidsegment transkriptionell an das wenigstens eine der ersten und der zweiten Polynucleotidsegmente gebunden ist.

44. Nucleinsäure-Konstrukt oder -Konstruktsystem nach Anspruch 41, wobei das wenigstens eine zusätzliche Polypeptidsegment über ein Linkersegment transkriptionell an das wenigstens eine der ersten und der zweiten Polynucleotidsegmente gebunden ist.

45. Nucleinsäure-Konstrukt oder -Konstruktsystem nach Anspruch 44, wobei die Linkersequenz aus der Gruppe bestehend aus IRES und einer Proteasenspaltungs-Erkennungssequenz ausgewählt ist.

46. Nucleinsäure-Konstrukt oder -Konstruktsystem nach Anspruch 41, wobei das wenigstens eine zusätzliche Polypeptidsegment mit dem wenigstens einen der ersten und der zweiten Polynucleotidsegmente translationsfusioniert ist.

47. Kit, umfassend ein Gestell, um wenigstens eine Röhre in Eingriff zu bringen, wobei die wenigstens eine Röhre umfasst:
(a) ein erstes Polynucleotid, das einen Zellproliferations-Wachstumsfaktor codiert; und
(b) ein zweites Polynucleotid, das einen Zelladhärenzfaktor codiert.

48. Kit nach Anspruch 47, wobei das erste Polynucleotid in einem ersten Nucleinsäure-Expressionskonstrukt ist und das zweite Polynucleotid in einem zweiten Nucleinsäure-Expressionskonstrukt ist.

49. Verfahren zur Herstellung genetisch transformierter Endothelzellen und/oder Eingeweidemuskelzellen, wobei das Verfahren umfasst:
Transformieren der Endothelzellen und/oder Eingeweidemuskelzellen, die aus einer Säugergewebequelle gewonnen wurden, um wenigstens einen Zellproliferations-Wachstumsfaktor und wenigstens einen Zelladhärenzfaktor zu exprimieren.

50. Künstliches Gefäßtransplantat nach Anspruch 1 zur Verwendung bei der Behandlung von Gefäßkrankheit bei einem Patienten, der Bedarf daran hat.

## Revendications

1. Greffe vasculaire artificielle comprenant un élément tubulaire synthétique ayant une surface luminale qui est recouverte d'une pluralité de cellules endothéliales et/ou de cellules musculaires lisses qui sont génétiquement transformées pour exprimer au moins un facteur de croissance stimulant la prolifération cellulaire et au moins un facteur d'adhésion cellulaire.

2. Greffe vasculaire artificielle selon la revendication 1, dans laquelle une première partie de ladite pluralité de cellules endothéliales et/ou de cellules musculaires lisses est génétiquement transformée pour exprimer ledit au moins un facteur de croissance stimulant la prolifération cellulaire, et en outre dans laquelle une seconde partie de ladite pluralité de cellules endothéliales et/ou de cellules musculaires lisses est génétiquement transformée pour exprimer ledit au moins un facteur d'adhésion cellulaire.

3. Greffe vasculaire artificielle selon la revendication 1, dans laquelle ladite surface luminale est constituée d'une substance sélectionnée dans le groupe consistant en du polytétrafluoroéthylène (PTFE), du polytétrafluoroéthylène expansé (PTFEe), des fibres de polyester et du Dacron^{™}.

4. Greffe vasculaire artificielle selon la revendication 4, dans laquelle ladite surface de section transversale interne dudit élément tubulaire synthétique est comprise dans une plage d'environ 7 à 700 mm².

5. Greffe vasculaire artificielle selon la revendication 1, dans laquelle ladite pluralité de cellules endothéliales est dérivée d'une source sélectionnée dans le groupe consistant en un segment d'une veine, des cellules progénitrices de moelle osseuse, des cellules souches du sang périphérique et des cellules endothéliales circulantes.

6. Greffe vasculaire artificielle selon la revendication 1, dans laquelle ladite pluralité de cellules endothéliales et/ou de cellules musculaires lisses est dérivée d'un donneur humain ou animal.

7. Greffe vasculaire artificielle selon la revendication 1, dans laquelle ladite pluralité de cellules endothéliales forme une monocouche confluente au niveau de ladite surface luminale interne.

8. Greffe vasculaire artificielle selon la revendication 1, dans laquelle ledit au moins un facteur de croissance stimulant la prolifération cellulaire est sélectionné dans le groupe consistant en le VEGF, le FGF acide ou basique et le HGF.

9. Greffe vasculaire artificielle selon la revendication 1, dans laquelle ledit au moins un facteur d'adhésion cellulaire est UP50, DANCE, la vitronectine, l'albumine, le collagène I, le collagène IV, la fibronectine et la laminine.

10. Greffe vasculaire artificielle selon la revendication 1, dans laquelle ladite pluralité de cellules endothéliales et/ou de cellules musculaires lisses sont en outre génétiquement transformées pour exprimer au moins un polypeptide marqueur.

11. Greffe vasculaire artificielle selon la revendication 1, dans laquelle ledit élément tubulaire synthétique ayant ladite surface luminale est recouvert de cellules endothéliales et de cellules musculaires lisses, au moins une desdites cellules endothéliales et cellules musculaires lisses étant génétiquement transformée pour exprimer au moins un facteur de croissance stimulant la prolifération cellulaire et au moins un facteur d'adhésion cellulaire.

12. Greffe vasculaire artificielle selon la revendication 1, dans laquelle ledit élément tubulaire synthétique ayant ladite surface luminale est recouvert d'une pluralité de cellules endothéliales qui sont génétiquement transformées pour exprimer UP50 et le VEGF.

13. Greffe vasculaire artificielle selon la revendication 1, dans laquelle ledit élément tubulaire synthétique ayant ladite surface luminale est recouvert d'une pluralité de cellules musculaires lisses qui sont génétiquement transformées pour exprimer UP50 et le VEGF.

14. Greffe vasculaire artificielle selon la revendication 1, dans laquelle ladite pluralité de cellules endothéliales et/ou de cellules musculaires lisses sont génétiquement transformées pour exprimer UP50 et un facteur de croissance stimulant la prolifération cellulaire sélectionné dans le groupe consistant en le FGF acide ou basique et le HGF.

15. Greffe vasculaire artificielle selon la revendication 1, dans laquelle ladite surface luminale est recouverte par ladite pluralité de cellules endothéliales et une surface externe dudit élément tubulaire synthétique est recouverte par ladite pluralité de cellules musculaires lisses.

16. Greffe vasculaire artificielle selon la revendication 15, dans laquelle ladite pluralité de cellules endothéliales est génétiquement transformée pour exprimer au moins un facteur de croissance stimulant la prolifération cellulaire et au moins un facteur d'adhésion cellulaire.

17. Greffe vasculaire artificielle selon la revendication 15, dans laquelle ladite pluralité de cellules musculaires lisses est génétiquement transformée pour exprimer au moins un facteur d'adhésion cellulaire.

18. Cellule endothéliale ou cellule musculaire lisse transformée qui est génétiquement transformée pour exprimer au moins un facteur de croissance stimulant la prolifération cellulaire et au moins un facteur d'adhésion cellulaire.

19. Cellule endothéliale ou cellule musculaire lisse transformée selon la revendication 18, qui est en outre génétiquement transformée pour exprimer au moins un polypeptide marqueur.

20. Cellule endothéliale ou cellule musculaire lisse transformée selon la revendication 18, où ladite cellule endothéliale est dérivée d'une source sélectionnée dans le groupe consistant en un segment d'une veine, et des cellules progénitrices de moelle osseuse, des cellules souches du sang périphérique et des cellules endothéliales circulantes.

21. Cellule endothéliale ou cellule musculaire lisse transformée selon la revendication 18, où ladite cellule endothéliale ou cellule musculaire lisse est dérivée d'une source sélectionnée dans le groupe consistant en un donneur humain ou une source animale.

22. Cellule endothéliale ou cellule musculaire lisse transformée selon la revendication 18, dans laquelle ledit facteur de croissance stimulant la prolifération cellulaire est sélectionné dans le groupe consistant en le VEGF, le FGF basique ou acide et le HGF.

23. Cellule endothéliale ou cellule musculaire lisse transformée selon la revendication 18, dans laquelle ledit facteur d'adhésion cellulaire est UP50, DANCE, la vitronectine, l'albumine, le collagène I, le collagène IV, la fibronectine et la laminine.

24. Procédé de production d'une greffe vasculaire artificielle, le procédé consistant à :
(a) génétiquement transformer des cellules endothéliales et/ou des cellules musculaires lisses afin d'exprimer au moins un facteur de croissance stimulant la prolifération cellulaire et au moins un facteur d'adhésion cellulaire ; et
(b) cultiver lesdites cellules endothéliales et/ou cellules musculaires lisses au sein d'un élément tubulaire synthétique ayant une surface luminale jusqu'à obtenir un recouvrement suffisant de ladite surface luminale par lesdites cellules endothéliales et/ou cellules musculaires lisses.

25. Procédé selon la revendication 24, dans lequel l'étape (a) est réalisée par la transformation génétique d'une première partie desdites cellules endothéliales et/ou cellules musculaires lisses afin d'exprimer ledit au moins un facteur de croissance stimulant la prolifération cellulaire et par la transformation génétique d'une seconde partie desdites cellules endothéliales et/ou cellules musculaires lisses afin d'exprimer ledit au moins un facteur d'adhésion cellulaire.

26. Procédé selon la revendication 24, dans lequel ladite étape (a) précède ladite étape (b).

27. Procédé selon la revendication 24, dans lequel ladite étape (b) précède ladite étape (a).

28. Procédé selon la revendication 24, dans lequel lesdites cellules endothéliales sont dérivées d'une source sélectionnée dans le groupe consistant en un segment d'une veine, et des cellules progénitrices de moelle osseuse, des cellules souches du sang périphérique et des cellules endothéliales circulantes.

29. Procédé selon la revendication 24, consistant en outre à soumettre ladite surface luminale de ladite greffe artificielle à des forces d'écoulement.

30. Procédé selon la revendication 24, dans lequel ledit au moins un facteur de croissance stimulant la prolifération cellulaire est le VEGF et ledit au moins un facteur d'adhésion cellulaire est UP50.

31. Produit de recombinaison d'expression d'acide nucléique ou système de produit de recombinaison comprenant :
(a) un premier segment polynucléotidique codant pour un facteur de croissance stimulant la prolifération cellulaire ; et
(b) un second segment polynucléotidique codant pour un facteur d'adhésion cellulaire.

32. Produit de recombinaison d'expression d'acide nucléique ou système de produit de recombinaison selon la revendication 31, comprenant en outre au moins une séquence promotrice qui est présente pour diriger l'expression d'au moins l'un dudit premier et dudit second segments polynucléotidiques.

33. Produit de recombinaison d'acide nucléique ou système de produit de recombinaison selon la revendication 32, dans lequel ledit premier segment polynucléotidique est lié de manière transcriptionnelle audit second segment polynucléotidique alors que ledit premier et ledit second segments polynucléotidiques sont sous le contrôle transcriptionnel d'une seule séquence promotrice de ladite au moins une séquence promotrice.

34. Produit de recombinaison d'acide nucléique ou système de produit de recombinaison selon la revendication 33, comprenant en outre une séquence de liaison qui est interposée entre ledit premier et ledit second segments polynucléotidiques.

35. Produit de recombinaison d'acide nucléique ou système de produit de recombinaison selon la revendication 34, dans lequel ladite séquence de liaison est sélectionnée dans le groupe consistant en un IRES et un site de reconnaissance de clivage par une protéase.

36. Produit de recombinaison d'expression d'acide nucléique ou système de produit de recombinaison selon la revendication 31, dans lequel ledit au moins un promoteur est fonctionnel dans des cellules eucaryotes.

37. Produit de recombinaison d'expression d'acide nucléique ou système de produit de recombinaison selon la revendication 31, dans lequel ledit au moins un promoteur est sélectionné dans le groupe consistant en un promoteur constitutif, un promoteur inductible et un promoteur spécifique à un tissu.

38. Produit de recombinaison d'expression d'acide nucléique ou système de produit de recombinaison selon la revendication 31, comprenant en outre :
(c) une première séquence promotrice qui est présente pour diriger l'expression dudit premier segment polynucléotidique ; et
(d) une seconde séquence promotrice qui est présente pour diriger l'expression dudit second segment polynucléotidique.

39. Produit de recombinaison d'expression d'acide nucléique ou système de produit de recombinaison selon la revendication 38, dans lequel ledit premier promoteur et ledit second promoteur sont sélectionnés dans le groupe consistant en un promoteur constitutif, un promoteur inductible et un promoteur spécifique à un tissu.

40. Produit de recombinaison d'expression d'acide nucléique ou système de produit de recombinaison selon la revendication 39, dans lequel lesdits promoteurs inductibles peuvent être régulés par la même molécule effectrice.

41. Produit de recombinaison d'expression d'acide nucléique ou système de produit de recombinaison selon la revendication 31, comprenant en outre au moins un segment polynucléotidique supplémentaire codant pour un polypeptide marqueur.

42. Produit de recombinaison d'expression d'acide nucléique ou système de produit de recombinaison selon la revendication 41, dans lequel ledit polypeptide marqueur est sélectionné dans le groupe consistant en un polypeptide de sélection et un polypeptide rapporteur.

43. Produit de recombinaison d'expression d'acide nucléique ou système de produit de recombinaison selon la revendication 41, dans lequel ledit au moins un segment polynucléotidique supplémentaire est lié de manière transcriptionnelle audit au moins un dudit premier et dudit second segments polynucléotidiques.

44. Produit de recombinaison d'acide nucléique ou système de produit de recombinaison selon la revendication 41, dans lequel ledit au moins un segment polynucléotidique supplémentaire est lié de manière transcriptionnelle audit au moins un dudit premier et dudit second segments polynucléotidiques via un segment de liaison.

45. Produit de recombinaison d'acide nucléique ou système de produit de recombinaison selon la revendication 44, dans lequel ladite séquence de liaison est sélectionnée dans le groupe consistant en un IRES et un site de reconnaissance de clivage par une protéase.

46. Produit de recombinaison d'acide nucléique ou système de produit de recombinaison selon la revendication 41, dans lequel ledit au moins un segment polynucléotidique supplémentaire est fusionné de manière traductionnelle à au moins un dudit premier et dudit second segments polynucléotidiques.

47. Kit comprenant un support pour engager au moins un tube, ledit au moins un tube comprenant :
(a) un premier polynucléotide codant pour un facteur de croissance stimulant la prolifération cellulaire ; et
(b) un second polynucléotide codant pour un facteur d'adhésion cellulaire.

48. Kit selon la revendication 47, dans lequel ledit premier polynucléotide est dans un premier produit de recombinaison d'expression d'acide nucléique et ledit second polynucléotide est dans un second produit de recombinaison d'expression d'acide nucléique.

49. Procédé pour produire des cellules endothéliales et/ou des cellules musculaires lisses génétiquement transformées, le procédé consistant à :
transformer lesdites cellules endothéliales et/ou cellules musculaires lisses qui ont été obtenues à partir d'une source tissulaire de mammifère, afin d'exprimer au moins un facteur de croissance stimulant la prolifération cellulaire et au moins un facteur d'adhésion cellulaire.

50. Greffe vasculaire artificielle selon la revendication 1, destinée à être utilisée dans le traitement d'une maladie vasculaire chez un sujet nécessitant celle-ci.


### SEQUENCE LISTING

<110> Flugelman, Moshe et al.
<120> ARTIFICIAL VASCULAR GRAFTS, AND METHODS OF PRODUCING AND USING SAME
<130> 01/22275
<150> 09/620,227
   <151> 2000-07-20
<160> 3
<170> PatentIn version 3.0
<210> 1
   <211> 1347
   <212> DNA
   <213> Homo sapiens
<400> 1
   atgccaggaa taaaaaggat actcactgtt accattctgg ctctctgtct tccaagccct 60
   gggaatgcac aggcacagtg cacgaatggc tttgacctgg atcgccagtc aggacagtgt 120
   ttagatattg atgaatgccg aaccatcccc gaggcctgcc gaggagacat gatgtgtgtt 180
   aaccaaaatg gcgggtattt atgcattccc cggacaaacc ctgtgtatcg agggccctac 240
   tcgaacccct actcgacccc ctactcaggt ccgtacccag cagctgcccc accactctca 300
   gctccaaact atcccacgat ctccaggcct cttatatgcc gctttggata ccagatggat 360
   gaaagcaacc aatgtgtgga tgtggacgag tgtgcaacag attcccacca gtgcaacccc 420
   acccagatct gcatcaatac tgaaggcggg tacacctgct cctgcaccga cggatattgg 480
   cttctggaag gccagtgctt agacattgat gaatgtcgct atggttactg ccagcagctc 540
   tgtgcgaatg ttcctggatc ctattcttgt acatgcaacc ctggttttac cctcaatgag 600
   gatggaaggt cttgccaaga tgtgaacgag tgtgccaccg agaacccctg cgtgcaaacc 660
   tgcgtcaaca cctacggctc tttcatctgc cgctgtgacc caggatatga acttgaggaa 720
   gatggcgttc attgcagtga tatggacgag tgcagcttct ctgagttcct ctgccaacat 780
   gagtgtgtga accagcccgg cacatacttc tgctcctgcc ctccaggcta catcctgctg 840
   gatgacaacc gaagctgcca agacatcaac gaatgtgagc acaggaacca cacgtgcaac 900
   ctgcagcaga cgtgctacaa tttacaaggg ggcttcaaat gcatcgaccc catccgctgt 960
   gaggagcctt atctgaggat cagtgataac cgctgtatgt gtcctgctga gaaccctggc 1020
   tgcagagacc agccctttac catcttgtac cgggacatgg acgtggtgtc aggacgctcc 1080
   gttcccgctg acatcttcca aatgcaagcc acgacccgct accctggggc ctattacatt 1140
   ttccagatca aatctgggaa tgagggcaga gaattttaca tgcggcaaac gggccccatc 1200
   agtgccaccc tggtgatgac acgccccatc aaagggcccc gggaaatcca gctggacttg 1260
   gaaatgatca ctgtcaacac tgtcatcaac ttcagaggca gctccgtgat ccgactgcgg 1320
   atatatgtgt cgcagtaccc attctga                                      1347
<210> 2
   <211> 36
   <212> DNA
   <213> Artificial
<220>
   <223> synthetic oligonucleotide
<400> 2
   gaagatcttg acatgccagg aataaaaagg atactc 36
<210> 3
   <211> 44
   <212> DNA
   <213> Artificial
<220>
   <223> synthetic oligonucleotide
<400> 3
   gaagatcttc agaatgggta ctgcgacaca tatatccgca gtcg 44